# DEMANDE DE BREVET EUROPEEN

(11) **EP 1 862 553 A2**
(43) Date de publication de la demande: **05.12.2007**
(21) Numéro de dépôt: 07115389.4
(22) Date de dépôt: 05.12.2001
(51) Int. Cl.: C12N 15/82, C12N 15/53, C12N 15/55, C12N 9/02, C12N 9/10, A01H 5/00

(54) **Nouvelles cibles pour herbicides et plantes transgéniques résistantes à ces herbicides**

(30) Priorité: 05.12.2000 FR 0015723
(62) Demande divisionnaire de: 01999658.6
(71) Demandeur: Bayer CropScience S.A., 69009 Lyon (FR)
(72) Inventeur: Matringe, Michel, 38100, GRENOBLE (FR); Rippert, Pascal, 8044, ZURICH (CH)
(74) Mandataire: Monconduit, Hervé

(57) **Abrégé**

La présente invention a pour objet de nouvelles enzymes possédant une activité arogénate déhydrogénase, en particulier des enzymes arogénate déhydrogénase de plantes, ainsi que les gènes codant ces enzymes. Les enzymes arogénate déhydrogénase selon l'invention catalysent la dernière étape de la voie métabolique de biosynthèse de la tyrosine, et constituent, à ce titre, des cibles potentielles d'herbicides. La présente invention concerne donc également un procédé d'identification de composés herbicides ayant pour cible ces enzymes, lesdits composés herbicides empêchant la biosynthèse de tyrosine en se fixant sur lesdites enzymes. L'invention a également pour objet des plantes transgénique tolérantes à des composés herbicides ayant pour cible une enzyme impliquée dans la voie de biosynthèse de la tyrosine, en particulier une enzyme impliquée dans la transformation du préphénate en L-tyrosine, en particulier une enzyme arogénate déhydrogénase. Ces plantes deviennent tolérantes par expression dans leurs tissus d'une enzyme préphénate dehydrogénase, cette enzyme étant insensible aux dits composés herbicides et permettant à la plante de synthétiser la tyrosine malgré un traitement par lesdits composés herbicides.

LISTE DE SEQUENCES

<110> Aventis CropScience SA

<120> Nouvelles cibles herbicides et plantes transgéniques
      résistantes à ces herbicides

<130> PRO00092

<140>
<141>

<160> 30

<170> PatentIn Ver. 2.1

<210> 1
<211> 2082
<212> ADN
<213> Arabidopsis thaliana

<220>
<221> transit_peptide
<222> (1)..(174)

<220>
<221> intron
<222> (1089)..(1181)

<400> 1
atgatctttc aatctcattc tcatcatctt cttctctatc aatcctcatc ttcctcctcc 60
ttcttcttcc tcccaaagct catcaccaaa cctcctctct ccctctcatt tacctctctt 120
tcctcaatgc tcccttctct ctctctctcc accgccaacc gccacctctc cgtcaccgac 180
accatccctc ttcccaactc caactccaac gccacccctc ctctccgtat cgccatcatc 240
ggattcggaa actacggcca attccttgcc gaaaccctaa tttctcaagg ccacattctc 300
ttcgctcact cccgatccga tcactcctcc gccgctcgcc gtctcggtgt ctcatacttc 360
accgatcttc acgatctctg cgaacgtcat cctgacgtag tccttctctg tacttcaatc 420
ctctccatag agaatattct caaaacgttg ccgtttcaga gactccgtcg caacactctc 480
ttcgttgatg ttctctccgt taaagagttt gctaaaactc ttctccttca atacttacct 540
gaagatttcg atattctttg tacacatcca atgtttggtc ctcagagtgt gagttcaaat 600
catggctgga gaggattaag atttgtgtat gataaagtta ggattgggga agagagattg 660
agagtctcaa ggtgtgagag ttttcttgag atttttgtta gagaaggatg tgagatggtg 720
gagatgagtg ttactgatca tgataagttt gctgctgaat cacagtttat aactcatact 780
cttggtaggc ttttggggat gttgaagttg atatcgacgc cgattaatac gaaagggtac 840
gaggcgttgc ttgatttagc tgagaatatt tgtggggata gttttgattt gtattatggg 900
ttgtttgtgt ataataacaa ctctttggag gtgttagaga ggattgattt ggctttcgag 960
gctttgcgta aggagctttt tagtcggctt cacggtgttg tgaggaagca gtcttttgaa 1020
ggtgaagcaa agaaagttca tgtttttcca aattgtggtg aaaatgatgc ttctttggat 1080
atgatgaggt atgtagttag ttagttagtt acattgtgtg gtttgatgca ttttggattt 1140
ggtttcttat tgtaaatagt tatcgatttg tgatcttgca ggtcagaaga tgttgttgtg 1200
aagtatgaat ataactccca ggtgtctggt agtgttaatg acggttcgag gctcaagatt 1260
ggtatcgtcg ggtttggaaa ttttggacag tttctaggta aaaccatggt caagcagggt 1320
cacactgtgt tagcttattc cagaagtgac tacactgatg aagcagcaaa gctcggtgtt 1380
tcgtattttt cagatcttga tgatctattt gaagagcatc ctgaagttat tattctctgt 1440
acgtcaatcc tttcgactga aaaagttctc gagtcactac cgtttcagag actgaagaga 1500
agcacacttt ttgtggatgt actctcagta aaagagttcc cgaggaattt atttcttcaa 1560
actctcccac aagattttga tattttgtgc acgcatccta tgtttgggcc agagagtggt 1620
aaaaatggat ggaacaatct tgcctttgtg tttgataagg ttaggattgg aatggatgat 1680
agaagaaaat cgaggtgtaa cagttttctt gatatttttg cccgtgaagg atgtcgtatg 1740
gtggagatgt cgtgtgctga acatgattgg catgctgctg gatcacagtt tatcacacac 1800
acagtgggaa ggcttctgga gaagctgagc ttggaatcta ctcctataga taccaaaggt 1860
tatgagacat tgctaaaact ggtggagaat actgctggtg acagctttga tctgtactat 1920
ggactatttt tatacaatcc taatgcaatg gaacagcttg agaggtttca tgtggctttt 1980
gaatcattga agacacagct ctttggacga ctacattctc aacattctca tgagctagct 2040

aaatcatctt ccccaaagac aactaagcta ttaactagct aa                    2082

<210> 2
<211> 1981
<212> ADN
<213> Arabidopsis thaliana

<220>
<221> CDS
<222> (1)..(1980)

<220>
<221> transit_peptide
<222> (1)..(174)

<400> 2
atg atc ttt caa tct cat tct cat cat ctt ctt ctc tat caa tcc tca    48
Met Ile Phe Gln Ser His Ser His His Leu Leu Leu Tyr Gln Ser Ser
1               5                   10                  15

tct tcc tcc tcc ttc ttc ttc ctc cca aag ctc atc acc aaa cct cct    96
Ser Ser Ser Ser Phe Phe Phe Leu Pro Lys Leu Ile Thr Lys Pro Pro
            20                  25                  30

ctc tcc ctc tca ttt acc tct ctt tcc tca atg ctc cct tct ctc tct    144
Leu Ser Leu Ser Phe Thr Ser Leu Ser Ser Met Leu Pro Ser Leu Ser
        35                  40                  45

ctc tcc acc gcc aac cgc cac ctc tcc gtc acc gac acc atc cct ctt    192
Leu Ser Thr Ala Asn Arg His Leu Ser Val Thr Asp Thr Ile Pro Leu
    50                  55                  60

ccc aac tcc aac tcc aac gcc acc cct cct ctc cgt atc gcc atc atc    240
Pro Asn Ser Asn Ser Asn Ala Thr Pro Pro Leu Arg Ile Ala Ile Ile
65                  70                  75                  80

gga ttc gga aac tac ggc caa ttc ctt gcc gaa acc cta att tct caa    288
Gly Phe Gly Asn Tyr Gly Gln Phe Leu Ala Glu Thr Leu Ile Ser Gln
                85                  90                  95

ggc cac att ctc ttc gct cac tcc cga tcc gat cac tcc tcc gcc gct    336
Gly His Ile Leu Phe Ala His Ser Arg Ser Asp His Ser Ser Ala Ala
            100                 105                 110

cgc cgt ctc ggt gtc tca tac ttc acc gat ctt cac gat ctc tgc gaa    384
Arg Arg Leu Gly Val Ser Tyr Phe Thr Asp Leu His Asp Leu Cys Glu
        115                 120                 125

cgt cat cct gac gta gtc ctt ctc tgt act tca atc ctc tcc ata gag    432
Arg His Pro Asp Val Val Leu Leu Cys Thr Ser Ile Leu Ser Ile Glu
    130                 135                 140

aat att ctc aaa acg ttg ccg ttt cag aga ctc cgt cgc aac act ctc    480
Asn Ile Leu Lys Thr Leu Pro Phe Gln Arg Leu Arg Arg Asn Thr Leu
145                 150                 155                 160

ttc gtt gat gtt ctc tcc gtt aaa gag ttt gct aaa act ctt ctc ctt    528
Phe Val Asp Val Leu Ser Val Lys Glu Phe Ala Lys Thr Leu Leu Leu
                165                 170                 175

caa tac tta cct gaa gat ttc gat att ctt tgt aca cat cca atg ttt    576
Gln Tyr Leu Pro Glu Asp Phe Asp Ile Leu Cys Thr His Pro Met Phe
            180                 185                 190

ggt cct cag agt gtg agt tca aat cat ggc tgg aga gga tta aga ttt    624
Gly Pro Gln Ser Val Ser Ser Asn His Gly Trp Arg Gly Leu Arg Phe
        195                 200                 205

gtg tat gat aaa gtt agg att ggg gaa gag aga ttg aga gtc tca agg    672
Val Tyr Asp Lys Val Arg Ile Gly Glu Glu Arg Leu Arg Val Ser Arg
    210                 215                 220

tgt gag agt ttt ctt gag att ttt gtt aga gaa gga tgt gag atg gtg    720
Cys Glu Ser Phe Leu Glu Ile Phe Val Arg Glu Gly Cys Glu Met Val
225                 230                 235                 240

gag atg agt gtt act gat cat gat aag ttt gct gct gaa tca cag ttt    768
Glu Met Ser Val Thr Asp His Asp Lys Phe Ala Ala Glu Ser Gln Phe
                245                 250                 255

ata act cat act ctt ggt agg ctt ttg ggg atg ttg aag ttg ata tcg    816
Ile Thr His Thr Leu Gly Arg Leu Leu Gly Met Leu Lys Leu Ile Ser
            260                 265                 270

acg ccg att aat acg aaa ggg tac gag gcg ttg ctt gat tta gct gag    864
Thr Pro Ile Asn Thr Lys Gly Tyr Glu Ala Leu Leu Asp Leu Ala Glu
        275                 280                 285

aat att tgt ggg gat agt ttt gat ttg tat tat ggg ttg ttt gtg tat    912
Asn Ile Cys Gly Asp Ser Phe Asp Leu Tyr Tyr Gly Leu Phe Val Tyr
    290                 295                 300

aat aac aac tct ttg gag gtg tta gag agg att gat ttg gct ttc gag    960
Asn Asn Asn Ser Leu Glu Val Leu Glu Arg Ile Asp Leu Ala Phe Glu
305                 310                 315                 320

gct ttg cgt aag gag ctt ttt agt cgg ctt cac ggt gtt gtg agg aag    1008
Ala Leu Arg Lys Glu Leu Phe Ser Arg Leu His Gly Val Val Arg Lys
                325                 330                 335

cag tct ttt gaa ggt gaa gca aag aaa gtt cat gtt ttt cca aat tgt    1056
Gln Ser Phe Glu Gly Glu Ala Lys Lys Val His Val Phe Pro Asn Cys
            340                 345                 350

ggt gaa aat gat gct tct ttg gat atg atg agg tca gaa gat gtt gtt    1104
Gly Glu Asn Asp Ala Ser Leu Asp Met Met Arg Ser Glu Asp Val Val
        355                 360                 365

gtg aag tat gaa tat aac tcc cag gtg tct ggt agt gtt aat gac ggt    1152
Val Lys Tyr Glu Tyr Asn Ser Gln Val Ser Gly Ser Val Asn Asp Gly
    370                 375                 380

tcg agg ctc aag att ggt atc gtc ggg ttt gga aat ttt gga cag ttt    1200
Ser Arg Leu Lys Ile Gly Ile Val Gly Phe Gly Asn Phe Gly Gln Phe
385                 390                 395                 400

cta ggt aaa acc atg gtc aag cag ggt cac act gtg tta gct tat tcc    1248
Leu Gly Lys Thr Met Val Lys Gln Gly His Thr Val Leu Ala Tyr Ser
                405                 410                 415

aga agt gac tac act gat gaa gca gca aag ctc ggt gtt tcg tat ttt    1296
Arg Ser Asp Tyr Thr Asp Glu Ala Ala Lys Leu Gly Val Ser Tyr Phe
            420                 425                 430

tca gat ctt gat gat cta ttt gaa gag cat cct gaa gtt att att ctc    1344
Ser Asp Leu Asp Asp Leu Phe Glu Glu His Pro Glu Val Ile Ile Leu
        435                 440                 445

tgt acg tca atc ctt tcg act gaa aaa gtt ctc gag tca cta ccg ttt    1392
Cys Thr Ser Ile Leu Ser Thr Glu Lys Val Leu Glu Ser Leu Pro Phe
    450                 455                 460

cag aga ctg aag aga agc aca ctt ttt gtg gat gta ctc tca gta aaa    1440
Gln Arg Leu Lys Arg Ser Thr Leu Phe Val Asp Val Leu Ser Val Lys
465                 470                 475                 480

gag ttc ccg agg aat tta ttt ctt caa act ctc cca caa gat ttt gat    1488
Glu Phe Pro Arg Asn Leu Phe Leu Gln Thr Leu Pro Gln Asp Phe Asp
                485                 490                 495

att ttg tgc acg cat cct atg ttt ggg cca gag agt ggt aaa aat gga    1536
Ile Leu Cys Thr His Pro Met Phe Gly Pro Glu Ser Gly Lys Asn Gly
            500                 505                 510

tgg aac aat ctt gcc ttt gtg ttt gat aag gtt agg att gga atg gat    1584
Trp Asn Asn Leu Ala Phe Val Phe Asp Lys Val Arg Ile Gly Met Asp
        515                 520                 525

gat aga aga aaa tcg agg tgt aac agt ttt ctt gat att ttt gcc cgt    1632
Asp Arg Arg Lys Ser Arg Cys Asn Ser Phe Leu Asp Ile Phe Ala Arg
    530                 535                 540

gaa gga tgt cgt atg gtg gag atg tcg tgt gct gaa cat gat tgg cat    1680
Glu Gly Cys Arg Met Val Glu Met Ser Cys Ala Glu His Asp Trp His
545                 550                 555                 560

gct gct gga tca cag ttt atc aca cac aca gtg gga agg ctt ctg gag    1728
Ala Ala Gly Ser Gln Phe Ile Thr His Thr Val Gly Arg Leu Leu Glu
                565                 570                 575

aag ctg agc ttg gaa tct act cct ata gat acc aaa ggt tat gag aca    1776
Lys Leu Ser Leu Glu Ser Thr Pro Ile Asp Thr Lys Gly Tyr Glu Thr
            580                 585                 590

ttg cta aaa ctg gtg gag aat act gct ggt gac agc ttt gat ctg tac    1824
Leu Leu Lys Leu Val Glu Asn Thr Ala Gly Asp Ser Phe Asp Leu Tyr
        595                 600                 605

tat gga cta ttt tta tac aat cct aat gca atg gaa cag ctt gag agg    1872
Tyr Gly Leu Phe Leu Tyr Asn Pro Asn Ala Met Glu Gln Leu Glu Arg
    610                 615                 620

ttt cat gtg gct ttt gaa tca ttg aag aca cag ctc ttt gga cga cta    1920
Phe His Val Ala Phe Glu Ser Leu Lys Thr Gln Leu Phe Gly Arg Leu
625                 630                 635                 640

cat tct caa cat tct cat gag cta gct aaa tca tct tcc cca aag aca    1968
His Ser Gln His Ser His Glu Leu Ala Lys Ser Ser Ser Pro Lys Thr
                645                 650                 655

act aag cta tta a                                                  1981
Thr Lys Leu Leu
            660

<210> 3
<211> 660
<212> PRT
<213> Arabidopsis thaliana

<400> 3

Met Ile Phe Gln Ser His Ser His His Leu Leu Leu Tyr Gln Ser Ser
1               5                   10                  15

Ser Ser Ser Ser Phe Phe Phe Leu Pro Lys Leu Ile Thr Lys Pro Pro
            20                  25                  30

Leu Ser Leu Ser Phe Thr Ser Leu Ser Ser Met Leu Pro Ser Leu Ser
        35                  40                  45

Leu Ser Thr Ala Asn Arg His Leu Ser Val Thr Asp Thr Ile Pro Leu
    50                  55                  60

Pro Asn Ser Asn Ser Asn Ala Thr Pro Pro Leu Arg Ile Ala Ile Ile
65                  70                  75                  80

Gly Phe Gly Asn Tyr Gly Gln Phe Leu Ala Glu Thr Leu Ile Ser Gln
                85                  90                  95

Gly His Ile Leu Phe Ala His Ser Arg Ser Asp His Ser Ser Ala Ala
            100                 105                 110

Arg Arg Leu Gly Val Ser Tyr Phe Thr Asp Leu His Asp Leu Cys Glu
        115                 120                 125

Arg His Pro Asp Val Val Leu Leu Cys Thr Ser Ile Leu Ser Ile Glu
    130                 135                 140

Asn Ile Leu Lys Thr Leu Pro Phe Gln Arg Leu Arg Arg Asn Thr Leu
145                 150                 155                 160

Phe Val Asp Val Leu Ser Val Lys Glu Phe Ala Lys Thr Leu Leu Leu
                165                 170                 175

Gln Tyr Leu Pro Glu Asp Phe Asp Ile Leu Cys Thr His Pro Met Phe
            180                 185                 190

Gly Pro Gln Ser Val Ser Ser Asn His Gly Trp Arg Gly Leu Arg Phe
        195                 200                 205

Val Tyr Asp Lys Val Arg Ile Gly Glu Glu Arg Leu Arg Val Ser Arg
    210                 215                 220

Cys Glu Ser Phe Leu Glu Ile Phe Val Arg Glu Gly Cys Glu Met Val
225                 230                 235                 240

Glu Met Ser Val Thr Asp His Asp Lys Phe Ala Ala Glu Ser Gln Phe
                245                 250                 255

Ile Thr His Thr Leu Gly Arg Leu Leu Gly Met Leu Lys Leu Ile Ser
            260                 265                 270

Thr Pro Ile Asn Thr Lys Gly Tyr Glu Ala Leu Leu Asp Leu Ala Glu
        275                 280                 285

Asn Ile Cys Gly Asp Ser Phe Asp Leu Tyr Tyr Gly Leu Phe Val Tyr
    290                 295                 300

Asn Asn Asn Ser Leu Glu Val Leu Glu Arg Ile Asp Leu Ala Phe Glu
305                 310                 315                 320

Ala Leu Arg Lys Glu Leu Phe Ser Arg Leu His Gly Val Val Arg Lys
                325                 330                 335

Gln Ser Phe Glu Gly Glu Ala Lys Lys Val His Val Phe Pro Asn Cys

340                 345                 350

Gly Glu Asn Asp Ala Ser Leu Asp Met Met Arg Ser Glu Asp Val Val
        355                 360                 365

Val Lys Tyr Glu Tyr Asn Ser Gln Val Ser Gly Ser Val Asn Asp Gly
    370                 375                 380

Ser Arg Leu Lys Ile Gly Ile Val Gly Phe Gly Asn Phe Gly Gln Phe
385                 390                 395                 400

Leu Gly Lys Thr Met Val Lys Gln Gly His Thr Val Leu Ala Tyr Ser
                405                 410                 415

Arg Ser Asp Tyr Thr Asp Glu Ala Ala Lys Leu Gly Val Ser Tyr Phe
            420                 425                 430

Ser Asp Leu Asp Asp Leu Phe Glu Glu His Pro Glu Val Ile Ile Leu
        435                 440                 445

Cys Thr Ser Ile Leu Ser Thr Glu Lys Val Leu Glu Ser Leu Pro Phe
    450                 455                 460

Gln Arg Leu Lys Arg Ser Thr Leu Phe Val Asp Val Leu Ser Val Lys
465                 470                 475                 480

Glu Phe Pro Arg Asn Leu Phe Leu Gln Thr Leu Pro Gln Asp Phe Asp
                485                 490                 495

Ile Leu Cys Thr His Pro Met Phe Gly Pro Glu Ser Gly Lys Asn Gly
            500                 505                 510

Trp Asn Asn Leu Ala Phe Val Phe Asp Lys Val Arg Ile Gly Met Asp
        515                 520                 525

Asp Arg Arg Lys Ser Arg Cys Asn Ser Phe Leu Asp Ile Phe Ala Arg
    530                 535                 540

Glu Gly Cys Arg Met Val Glu Met Ser Cys Ala Glu His Asp Trp His
545                 550                 555                 560

Ala Ala Gly Ser Gln Phe Ile Thr His Thr Val Gly Arg Leu Leu Glu
                565                 570                 575

Lys Leu Ser Leu Glu Ser Thr Pro Ile Asp Thr Lys Gly Tyr Glu Thr
            580                 585                 590

Leu Leu Lys Leu Val Glu Asn Thr Ala Gly Asp Ser Phe Asp Leu Tyr
        595                 600                 605

Tyr Gly Leu Phe Leu Tyr Asn Pro Asn Ala Met Glu Gln Leu Glu Arg
    610                 615                 620

Phe His Val Ala Phe Glu Ser Leu Lys Thr Gln Leu Phe Gly Arg Leu
625                 630                 635                 640

His Ser Gln His Ser His Glu Leu Ala Lys Ser Ser Ser Pro Lys Thr
                645                 650                 655

Thr Lys Leu Leu
            660

<210> 4
<211> 933
<212> ADN
<213> Arabidopsis thaliana

<220>
<221> CDS
<222> (1)..(933)

<400> 4
atg acc gac acc atc cct ctt ccc aac tcc aac tcc aac gcc acc cct     48
Met Thr Asp Thr Ile Pro Leu Pro Asn Ser Asn Ser Asn Ala Thr Pro
1               5                   10                  15

cct ctc cgt atc gcc atc atc gga ttc gga aac tac ggc caa ttc ctt     96
Pro Leu Arg Ile Ala Ile Ile Gly Phe Gly Asn Tyr Gly Gln Phe Leu
            20                  25                  30

gcc gaa acc cta att tct caa ggc cac att ctc ttc gct cac tcc cga     144
Ala Glu Thr Leu Ile Ser Gln Gly His Ile Leu Phe Ala His Ser Arg
        35                  40                  45

tcc gat cac tcc tcc gcc gct cgc cgt ctc ggt gtc tca tac ttc acc     192
Ser Asp His Ser Ser Ala Ala Arg Arg Leu Gly Val Ser Tyr Phe Thr
    50                  55                  60

gat ctt cac gat ctc tgc gaa cgt cat cct gac gta gtc ctt ctc tgt     240
Asp Leu His Asp Leu Cys Glu Arg His Pro Asp Val Val Leu Leu Cys
65                  70                  75                  80

act tca atc ctc tcc ata gag aat att ctc aaa acg ttg ccg ttt cag     288
Thr Ser Ile Leu Ser Ile Glu Asn Ile Leu Lys Thr Leu Pro Phe Gln
                85                  90                  95

aga ctc cgt cgc aac act ctc ttc gtt gat gtt ctc tcc gtt aaa gag     336
Arg Leu Arg Arg Asn Thr Leu Phe Val Asp Val Leu Ser Val Lys Glu
            100                 105                 110

ttt gct aaa act ctt ctc ctt caa tac tta cct gaa gat ttc gat att     384
Phe Ala Lys Thr Leu Leu Leu Gln Tyr Leu Pro Glu Asp Phe Asp Ile
        115                 120                 125

ctt tgt aca cat cca atg ttt ggt cct cag agt gtg agt tca aat cat     432
Leu Cys Thr His Pro Met Phe Gly Pro Gln Ser Val Ser Ser Asn His
    130                 135                 140

ggc tgg aga gga tta aga ttt gtg tat gat aaa gtt agg att ggg gaa     480
Gly Trp Arg Gly Leu Arg Phe Val Tyr Asp Lys Val Arg Ile Gly Glu
145                 150                 155                 160

gag aga ttg aga gtc tca agg tgt gag agt ttt ctt gag att ttt gtt     528
Glu Arg Leu Arg Val Ser Arg Cys Glu Ser Phe Leu Glu Ile Phe Val
                165                 170                 175

aga gaa gga tgt gag atg gtg gag atg agt gtt act gat cat gat aag     576
Arg Glu Gly Cys Glu Met Val Glu Met Ser Val Thr Asp His Asp Lys
            180                 185                 190

ttt gct gct gaa tca cag ttt ata act cat act ctt ggt agg ctt ttg     624
Phe Ala Ala Glu Ser Gln Phe Ile Thr His Thr Leu Gly Arg Leu Leu
        195                 200                 205

ggg atg ttg aag ttg ata tcg acg ccg att aat acg aaa ggg tac gag     672
Gly Met Leu Lys Leu Ile Ser Thr Pro Ile Asn Thr Lys Gly Tyr Glu

210                 215                 220

gcg ttg ctt gat tta gct gag aat att tgt ggg gat agt ttt gat ttg     720
Ala Leu Leu Asp Leu Ala Glu Asn Ile Cys Gly Asp Ser Phe Asp Leu
225                 230                 235                 240

tat tat ggg ttg ttt gtg tat aat aac aac tct ttg gag gtg tta gag     768
Tyr Tyr Gly Leu Phe Val Tyr Asn Asn Asn Ser Leu Glu Val Leu Glu
                245                 250                 255

agg att gat ttg gct ttc gag gct ttg cgt aag gag ctt ttt agt cgg     816
Arg Ile Asp Leu Ala Phe Glu Ala Leu Arg Lys Glu Leu Phe Ser Arg
            260                 265                 270

ctt cac ggt gtt gtg agg aag cag tct ttt gaa ggt gaa gca aag aaa     864
Leu His Gly Val Val Arg Lys Gln Ser Phe Glu Gly Glu Ala Lys Lys
        275                 280                 285

gtt cat gtt ttt cca aat tgt ggt gaa aat gat gct tct ttg gat atg     912
Val His Val Phe Pro Asn Cys Gly Glu Asn Asp Ala Ser Leu Asp Met
    290                 295                 300

atg agg tat gta gtt agt tag                                         933
Met Arg Tyr Val Val Ser
305                 310


<210> 5
<211> 310
<212> PRT
<213> Arabidopsis thaliana

<400> 5
Met Thr Asp Thr Ile Pro Leu Pro Asn Ser Asn Ser Asn Ala Thr Pro
1               5                   10                  15
Pro Leu Arg Ile Ala Ile Ile Gly Phe Gly Asn Tyr Gly Gln Phe Leu
            20                  25                  30
Ala Glu Thr Leu Ile Ser Gln Gly His Ile Leu Phe Ala His Ser Arg
        35                  40                  45
Ser Asp His Ser Ser Ala Ala Arg Arg Leu Gly Val Ser Tyr Phe Thr
    50                  55                  60
Asp Leu His Asp Leu Cys Glu Arg His Pro Asp Val Val Leu Leu Cys
65                  70                  75                  80
Thr Ser Ile Leu Ser Ile Glu Asn Ile Leu Lys Thr Leu Pro Phe Gln
                85                  90                  95
Arg Leu Arg Arg Asn Thr Leu Phe Val Asp Val Leu Ser Val Lys Glu
            100                 105                 110
Phe Ala Lys Thr Leu Leu Leu Gln Tyr Leu Pro Glu Asp Phe Asp Ile
        115                 120                 125
Leu Cys Thr His Pro Met Phe Gly Pro Gln Ser Val Ser Ser Asn His
    130                 135                 140
Gly Trp Arg Gly Leu Arg Phe Val Tyr Asp Lys Val Arg Ile Gly Glu
145                 150                 155                 160
Glu Arg Leu Arg Val Ser Arg Cys Glu Ser Phe Leu Glu Ile Phe Val
                165                 170                 175
Arg Glu Gly Cys Glu Met Val Glu Met Ser Val Thr Asp His Asp Lys
            180                 185                 190
Phe Ala Ala Glu Ser Gln Phe Ile Thr His Thr Leu Gly Arg Leu Leu
        195                 200                 205
Gly Met Leu Lys Leu Ile Ser Thr Pro Ile Asn Thr Lys Gly Tyr Glu
    210                 215                 220
Ala Leu Leu Asp Leu Ala Glu Asn Ile Cys Gly Asp Ser Phe Asp Leu
225                 230                 235                 240
Tyr Tyr Gly Leu Phe Val Tyr Asn Asn Asn Ser Leu Glu Val Leu Glu

245                 250                 255
Arg Ile Asp Leu Ala Phe Glu Ala Leu Arg Lys Glu Leu Phe Ser Arg
            260                 265                 270
Leu His Gly Val Val Arg Lys Gln Ser Phe Glu Gly Glu Ala Lys Lys
        275                 280                 285
Val His Val Phe Pro Asn Cys Gly Glu Asn Asp Ala Ser Leu Asp Met
    290                 295                 300
Met Arg Tyr Val Val Ser
305                 310

<210> 6
<211> 909
<212> ADN
<213> Arabidopsis thaliana

<220>
<221> CDS
<222> (1)..(909)

<400> 6
atg atg agg tca gaa gat gtt gtt gtg aag tat gaa tat aac tcc cag      48
Met Met Arg Ser Glu Asp Val Val Val Lys Tyr Glu Tyr Asn Ser Gln
1               5                   10                  15

gtg tct ggt agt gtt aat gac ggt tcg agg ctc aag att ggt atc gtc      96
Val Ser Gly Ser Val Asn Asp Gly Ser Arg Leu Lys Ile Gly Ile Val
            20                  25                  30

ggg ttt gga aat ttt gga cag ttt cta ggt aaa acc atg gtc aag cag     144
Gly Phe Gly Asn Phe Gly Gln Phe Leu Gly Lys Thr Met Val Lys Gln
        35                  40                  45

ggt cac act gtg tta gct tat tcc aga agt gac tac act gat gaa gca     192
Gly His Thr Val Leu Ala Tyr Ser Arg Ser Asp Tyr Thr Asp Glu Ala
    50                  55                  60

gca aag ctc ggt gtt tcg tat ttt tca gat ctt gat gat cta ttt gaa     240
Ala Lys Leu Gly Val Ser Tyr Phe Ser Asp Leu Asp Asp Leu Phe Glu
65                  70                  75                  80

gag cat cct gaa gtt att att ctc tgt acg tca atc ctt tcg act gaa     288
Glu His Pro Glu Val Ile Ile Leu Cys Thr Ser Ile Leu Ser Thr Glu
                85                  90                  95

aaa gtt ctc gag tca cta ccg ttt cag aga ctg aag aga agc aca ctt     336
Lys Val Leu Glu Ser Leu Pro Phe Gln Arg Leu Lys Arg Ser Thr Leu
            100                 105                 110

ttt gtg gat gta ctc tca gta aaa gag ttc ccg agg aat tta ttt ctt     384
Phe Val Asp Val Leu Ser Val Lys Glu Phe Pro Arg Asn Leu Phe Leu
        115                 120                 125

caa act ctc cca caa gat ttt gat att ttg tgc acg cat cct atg ttt     432
Gln Thr Leu Pro Gln Asp Phe Asp Ile Leu Cys Thr His Pro Met Phe
    130                 135                 140

ggg cca gag agt ggt aaa aat gga tgg aac aat ctt gcc ttt gtg ttt     480
Gly Pro Glu Ser Gly Lys Asn Gly Trp Asn Asn Leu Ala Phe Val Phe
145                 150                 155                 160

gat aag gtt agg att gga atg gat gat aga aga aaa tcg agg tgt aac     528
Asp Lys Val Arg Ile Gly Met Asp Asp Arg Arg Lys Ser Arg Cys Asn

165                 170                 175

agt ttt ctt gat att ttt gcc cgt gaa gga tgt cgt atg gtg gag atg     576
Ser Phe Leu Asp Ile Phe Ala Arg Glu Gly Cys Arg Met Val Glu Met
            180                 185                 190

tcg tgt gct gaa cat gat tgg cat gct gct gga tca cag ttt atc aca     624
Ser Cys Ala Glu His Asp Trp His Ala Ala Gly Ser Gln Phe Ile Thr
        195                 200                 205

cac aca gtg gga agg ctt ctg gag aag ctg agc ttg gaa tct act cct     672
His Thr Val Gly Arg Leu Leu Glu Lys Leu Ser Leu Glu Ser Thr Pro
    210                 215                 220

ata gat acc aaa ggt tat gag aca ttg cta aaa ctg gtg gag aat act     720
Ile Asp Thr Lys Gly Tyr Glu Thr Leu Leu Lys Leu Val Glu Asn Thr
225                 230                 235                 240

gct ggt gac agc ttt gat ctg tac tat gga cta ttt tta tac aat cct     768
Ala Gly Asp Ser Phe Asp Leu Tyr Tyr Gly Leu Phe Leu Tyr Asn Pro
                245                 250                 255

aat gca atg gaa cag ctt gag agg ttt cat gtg gct ttt gaa tca ttg     816
Asn Ala Met Glu Gln Leu Glu Arg Phe His Val Ala Phe Glu Ser Leu
            260                 265                 270

aag aca cag ctc ttt gga cga cta cat tct caa cat tct cat gag cta     864
Lys Thr Gln Leu Phe Gly Arg Leu His Ser Gln His Ser His Glu Leu
        275                 280                 285

gct aaa tca tct tcc cca aag aca act aag cta tta act agc taa         909
Ala Lys Ser Ser Ser Pro Lys Thr Thr Lys Leu Leu Thr Ser
    290                 295                 300


<210> 7
<211> 302
<212> PRT
<213> Arabidopsis thaliana

<400> 7
Met Met Arg Ser Glu Asp Val Val Val Lys Tyr Glu Tyr Asn Ser Gln
1               5                   10                  15
Val Ser Gly Ser Val Asn Asp Gly Ser Arg Leu Lys Ile Gly Ile Val
            20                  25                  30
Gly Phe Gly Asn Phe Gly Gln Phe Leu Gly Lys Thr Met Val Lys Gln
        35                  40                  45
Gly His Thr Val Leu Ala Tyr Ser Arg Ser Asp Tyr Thr Asp Glu Ala
    50                  55                  60
Ala Lys Leu Gly Val Ser Tyr Phe Ser Asp Leu Asp Asp Leu Phe Glu
65                  70                  75                  80
Glu His Pro Glu Val Ile Ile Leu Cys Thr Ser Ile Leu Ser Thr Glu
                85                  90                  95
Lys Val Leu Glu Ser Leu Pro Phe Gln Arg Leu Lys Arg Ser Thr Leu
            100                 105                 110
Phe Val Asp Val Leu Ser Val Lys Glu Phe Pro Arg Asn Leu Phe Leu
        115                 120                 125
Gln Thr Leu Pro Gln Asp Phe Asp Ile Leu Cys Thr His Pro Met Phe
    130                 135                 140
Gly Pro Glu Ser Gly Lys Asn Gly Trp Asn Asn Leu Ala Phe Val Phe
145                 150                 155                 160
Asp Lys Val Arg Ile Gly Met Asp Asp Arg Arg Lys Ser Arg Cys Asn
                165                 170                 175
Ser Phe Leu Asp Ile Phe Ala Arg Glu Gly Cys Arg Met Val Glu Met

                180                 185                 190
Ser Cys Ala Glu His Asp Trp His Ala Ala Gly Ser Gln Phe Ile Thr
        195                 200                 205
His Thr Val Gly Arg Leu Leu Glu Lys Leu Ser Leu Glu Ser Thr Pro
    210                 215                 220
Ile Asp Thr Lys Gly Tyr Glu Thr Leu Leu Lys Leu Val Glu Asn Thr
225                 230                 235                 240
Ala Gly Asp Ser Phe Asp Leu Tyr Tyr Gly Leu Phe Leu Tyr Asn Pro
                245                 250                 255
Asn Ala Met Glu Gln Leu Glu Arg Phe His Val Ala Phe Glu Ser Leu
            260                 265                 270
Lys Thr Gln Leu Phe Gly Arg Leu His Ser Gln His Ser His Glu Leu
        275                 280                 285
Ala Lys Ser Ser Ser Pro Lys Thr Thr Lys Leu Leu Thr Ser
    290                 295                 300

<210> 8
<211> 1077
<212> ADN
<213> Arabidopsis thaliana

<220>
<221> CDS
<222> (1)..(1077)

<400> 8
atg cta ctc cat ttc tct ccg gcg aaa ccc ctc att tct cca ccc aat     48
Met Leu Leu His Phe Ser Pro Ala Lys Pro Leu Ile Ser Pro Pro Asn
1               5                   10                  15

ctc cgc cgc aat tca ccc aca ttc ctc att tcc ccg ccg cga tct ctt     96
Leu Arg Arg Asn Ser Pro Thr Phe Leu Ile Ser Pro Pro Arg Ser Leu
            20                  25                  30

cga att cga gca atc gac gcc gcc caa atc ttc gat tac gaa acc caa     144
Arg Ile Arg Ala Ile Asp Ala Ala Gln Ile Phe Asp Tyr Glu Thr Gln
        35                  40                  45

ctc aaa tcc gag tac cgc aaa tcc tct gct ctc aaa atc gcc gtc ttg     192
Leu Lys Ser Glu Tyr Arg Lys Ser Ser Ala Leu Lys Ile Ala Val Leu
    50                  55                  60

ggt ttc ggc aat ttc ggc caa ttc ctc tcc aaa acc cta att cga cac     240
Gly Phe Gly Asn Phe Gly Gln Phe Leu Ser Lys Thr Leu Ile Arg His
65                  70                  75                  80

ggc cac gat cta atc act cac tcc cgc tcc gat tac tcc gac gcc gca     288
Gly His Asp Leu Ile Thr His Ser Arg Ser Asp Tyr Ser Asp Ala Ala
                85                  90                  95

aac tca atc gga gct cgt ttc ttc gat aac cct cac gat ctc tgt gaa     336
Asn Ser Ile Gly Ala Arg Phe Phe Asp Asn Pro His Asp Leu Cys Glu
            100                 105                 110

caa cat ccc gac gtt gtc ctc ctc tgt acc tca atc ctc tcc aca gaa     384
Gln His Pro Asp Val Val Leu Leu Cys Thr Ser Ile Leu Ser Thr Glu
        115                 120                 125

tca gtc ctc aga tca ttc cct ttc caa cgt ctc cgt cgt agc aca ctc     432
Ser Val Leu Arg Ser Phe Pro Phe Gln Arg Leu Arg Arg Ser Thr Leu
    130                 135                 140

ttc gtc gat gtt ctc tcc gtt aag gaa ttc cca aaa gcc ctc ttc att     480
Phe Val Asp Val Leu Ser Val Lys Glu Phe Pro Lys Ala Leu Phe Ile
145                 150                 155                 160

aaa tac ctt cct aag gag ttt gac att ctc tgt act cat cca atg ttt     528
Lys Tyr Leu Pro Lys Glu Phe Asp Ile Leu Cys Thr His Pro Met Phe
                165                 170                 175

gga cct gag agt ggt aag cat tct tgg tct ggc ttg ccc ttt gtc tac     576
Gly Pro Glu Ser Gly Lys His Ser Trp Ser Gly Leu Pro Phe Val Tyr
            180                 185                 190

gat aag gtg aga atc gga gac gca gct tca aga caa gag agg tgt gag     624
Asp Lys Val Arg Ile Gly Asp Ala Ala Ser Arg Gln Glu Arg Cys Glu
        195                 200                 205

aag ttt cta aga att ttt gag aat gaa ggt tgc aag atg gtt gaa atg     672
Lys Phe Leu Arg Ile Phe Glu Asn Glu Gly Cys Lys Met Val Glu Met
    210                 215                 220

agc tgt gag aag cat gat tat tac gca gct gga tcg caa ttc gtg acg     720
Ser Cys Glu Lys His Asp Tyr Tyr Ala Ala Gly Ser Gln Phe Val Thr
225                 230                 235                 240

cat act atg gga agg gtt ttg gag aaa tat gga gtt gag tct tcg ccg     768
His Thr Met Gly Arg Val Leu Glu Lys Tyr Gly Val Glu Ser Ser Pro
                245                 250                 255

att aac acc aaa ggt tat gag acg ttg ttg gat ttg gtg gag aac aca     816
Ile Asn Thr Lys Gly Tyr Glu Thr Leu Leu Asp Leu Val Glu Asn Thr
            260                 265                 270

tcg agt gat agc ttt gag ctt ttc tac ggt ttg ttt atg tat aat ccg     864
Ser Ser Asp Ser Phe Glu Leu Phe Tyr Gly Leu Phe Met Tyr Asn Pro
        275                 280                 285

aat gct ctt gaa cag ttg gag aga ttg gat atg gct ttt gag tct gtt     912
Asn Ala Leu Glu Gln Leu Glu Arg Leu Asp Met Ala Phe Glu Ser Val
    290                 295                 300

aag aag gag ctg ttt ggg aga tta cat cag caa tac agg aag caa atg     960
Lys Lys Glu Leu Phe Gly Arg Leu His Gln Gln Tyr Arg Lys Gln Met
305                 310                 315                 320

ttt ggt ggg gag gtt caa tcg ccc aag aaa act gag cag aaa ttg ctc     1008
Phe Gly Gly Glu Val Gln Ser Pro Lys Lys Thr Glu Gln Lys Leu Leu
                325                 330                 335

aat gat ggt ggt gtt gtt cct atg aat gat ata tca tca tca tca tca     1056
Asn Asp Gly Gly Val Val Pro Met Asn Asp Ile Ser Ser Ser Ser Ser
            340                 345                 350

tca tca tca tca tca tct taa                                         1077
Ser Ser Ser Ser Ser Ser
        355


<210> 9
<211> 358
<212> PRT
<213> Arabidopsis thaliana

<400> 9
Met Leu Leu His Phe Ser Pro Ala Lys Pro Leu Ile Ser Pro Pro Asn

1               5                   10                  15
Leu Arg Arg Asn Ser Pro Thr Phe Leu Ile Ser Pro Pro Arg Ser Leu
            20                  25                  30
Arg Ile Arg Ala Ile Asp Ala Ala Gln Ile Phe Asp Tyr Glu Thr Gln
        35                  40                  45
Leu Lys Ser Glu Tyr Arg Lys Ser Ser Ala Leu Lys Ile Ala Val Leu
    50                  55                  60
Gly Phe Gly Asn Phe Gly Gln Phe Leu Ser Lys Thr Leu Ile Arg His
65                  70                  75                  80
Gly His Asp Leu Ile Thr His Ser Arg Ser Asp Tyr Ser Asp Ala Ala
                85                  90                  95
Asn Ser Ile Gly Ala Arg Phe Phe Asp Asn Pro His Asp Leu Cys Glu
            100                 105                 110
Gln His Pro Asp Val Val Leu Leu Cys Thr Ser Ile Leu Ser Thr Glu
        115                 120                 125
Ser Val Leu Arg Ser Phe Pro Phe Gln Arg Leu Arg Arg Ser Thr Leu
    130                 135                 140
Phe Val Asp Val Leu Ser Val Lys Glu Phe Pro Lys Ala Leu Phe Ile
145                 150                 155                 160
Lys Tyr Leu Pro Lys Glu Phe Asp Ile Leu Cys Thr His Pro Met Phe
                165                 170                 175
Gly Pro Glu Ser Gly Lys His Ser Trp Ser Gly Leu Pro Phe Val Tyr
            180                 185                 190
Asp Lys Val Arg Ile Gly Asp Ala Ala Ser Arg Gln Glu Arg Cys Glu
        195                 200                 205
Lys Phe Leu Arg Ile Phe Glu Asn Glu Gly Cys Lys Met Val Glu Met
    210                 215                 220
Ser Cys Glu Lys His Asp Tyr Tyr Ala Ala Gly Ser Gln Phe Val Thr
225                 230                 235                 240
His Thr Met Gly Arg Val Leu Glu Lys Tyr Gly Val Glu Ser Ser Pro
                245                 250                 255
Ile Asn Thr Lys Gly Tyr Glu Thr Leu Leu Asp Leu Val Glu Asn Thr
            260                 265                 270
Ser Ser Asp Ser Phe Glu Leu Phe Tyr Gly Leu Phe Met Tyr Asn Pro
        275                 280                 285
Asn Ala Leu Glu Gln Leu Glu Arg Leu Asp Met Ala Phe Glu Ser Val
    290                 295                 300
Lys Lys Glu Leu Phe Gly Arg Leu His Gln Gln Tyr Arg Lys Gln Met
305                 310                 315                 320
Phe Gly Gly Glu Val Gln Ser Pro Lys Lys Thr Glu Gln Lys Leu Leu
                325                 330                 335
Asn Asp Gly Gly Val Val Pro Met Asn Asp Ile Ser Ser Ser Ser Ser
            340                 345                 350
Ser Ser Ser Ser Ser Ser
        355

<210> 10
<211> 1476
<212> ADN
<213> Picea glauca

<220>
<221> CDS
<222> (96)..(980)

<400> 10
accagtttta gatattcatc aaggtcttgc ctgctttgtt ttaggcaatt ccctccagta 60

ccaagccctc ttctcagaaa actccctccg cggca atg cct ctt cat ttc tca    113
                                       Met Pro Leu His Phe Ser
                                       1               5

tgg aat cca aca gaa gac cct cac aca gta cgc cct act gag gct ctc     161
Trp Asn Pro Thr Glu Asp Pro His Thr Val Arg Pro Thr Glu Ala Leu
            10                  15                  20

agg aat cag agc aat gga cgt cgc ggg gcc cct cga tta aga aga ata     209
Arg Asn Gln Ser Asn Gly Arg Arg Gly Ala Pro Arg Leu Arg Arg Ile
        25                  30                  35

aaa tcc att aaa tat tgg cat cgt agg gtt tgg aaa cta cca cca att     257
Lys Ser Ile Lys Tyr Trp His Arg Arg Val Trp Lys Leu Pro Pro Ile
    40                  45                  50

tct ggt gaa aac cat ggt gaa gcc ggg cca ccc ggt gct cgc cca ttc     305
Ser Gly Glu Asn His Gly Glu Ala Gly Pro Pro Gly Ala Arg Pro Phe
55                  60                  65                  70

cag gac gga cta tac gga ggc cac tgc gag atc ggg gtt caa ttc ttc     353
Gln Asp Gly Leu Tyr Gly Gly His Cys Glu Ile Gly Val Gln Phe Phe
                75                  80                  85

aga gac gcg gac gat ttc tgc gaa gag cat cca gag atc ata ctg atg     401
Arg Asp Ala Asp Asp Phe Cys Glu Glu His Pro Glu Ile Ile Leu Met
            90                  95                  100

tgc gca tcc atc act ttg gtg gga gga cgt gct gaa gtc tct gcc aac     449
Cys Ala Ser Ile Thr Leu Val Gly Gly Arg Ala Glu Val Ser Ala Asn
        105                 110                 115

aca gcg cct gaa gag gag tac gct ttt cgc aga cgt cct gtc tgt gaa     497
Thr Ala Pro Glu Glu Glu Tyr Ala Phe Arg Arg Arg Pro Val Cys Glu
    120                 125                 130

aga gtt tcc gca ccg gtt gtt cct gca ggt ttt gtc gcc cga gtc gat     545
Arg Val Ser Ala Pro Val Val Pro Ala Gly Phe Val Ala Arg Val Asp
135                 140                 145                 150

gtg ctg tgc act cat ccc atg ttt ggt cca gag agc agc aag gac gat     593
Val Leu Cys Thr His Pro Met Phe Gly Pro Glu Ser Ser Lys Asp Asp
                155                 160                 165

ttg ggc gac ctc cct ttc gtt tac gat aag gtt agg gtt tct aac gaa     641
Leu Gly Asp Leu Pro Phe Val Tyr Asp Lys Val Arg Val Ser Asn Glu
            170                 175                 180

ggt ttg aga gcc aag cac tgc gag cgt ttt ctc aac ata ttt tcg tgc     689
Gly Leu Arg Ala Lys His Cys Glu Arg Phe Leu Asn Ile Phe Ser Cys
        185                 190                 195

gag ggc tgc cgg atg gtc gag atg tcg tgt gca gaa cat gat cgc tat     737
Glu Gly Cys Arg Met Val Glu Met Ser Cys Ala Glu His Asp Arg Tyr
    200                 205                 210

gtc gcg gag agc caa ttc att acc cac acc gtt ggg agg atg ttg ggg     785
Val Ala Glu Ser Gln Phe Ile Thr His Thr Val Gly Arg Met Leu Gly
215                 220                 225                 230

agg ctg ggc ttg gag tcc act ccg att gct acc aag ggt tat gag aaa     833
Arg Leu Gly Leu Glu Ser Thr Pro Ile Ala Thr Lys Gly Tyr Glu Lys
                235                 240                 245

tta ctg gaa gtg gcc tgg aat att gcc ggg gat agt ttt gat att tat     881
Leu Leu Glu Val Ala Trp Asn Ile Ala Gly Asp Ser Phe Asp Ile Tyr
            250                 255                 260

tat gga ctc ttc atg tat aat gtc aat tcg att gaa caa atc gag agg     929
Tyr Gly Leu Phe Met Tyr Asn Val Asn Ser Ile Glu Gln Ile Glu Arg
        265                 270                 275

tta gat atg gcg ttc aat tca ctc aag aac gag gtt tcg ggt tca aat     977
Leu Asp Met Ala Phe Asn Ser Leu Lys Asn Glu Val Ser Gly Ser Asn
    280                 285                 290

taa gaattttaaa ggtttcgatt tgcttgaagc ggcttgtgta tgaagtacca        1030

295

tttgtagaca ataatgaatt cgagaatgtt gttcaagatg aaatggttaa gaaggatggg 1090

tctcgtgtga gaagaaaacc aagataaatg gttgcgtagt ggtccagaaa tctgcattca 1150

ttactgaatg attctacatg gagtgagtaa gcattgattg aattccaaga cgagtgaaag 1210

agtttgatga atggaatatg tctgtattcc aaatttaata aatgaaaaat attgcaggtt 1270

gctatatgca atggttcttc tatatccccg aaggacaaat gacagatata agttctctgg 1330

cacttgtcag aaaacttcta tgtttgtagc cataaaacat tttccgaaag tggaactttt 1390

ctagaactta taggggaaat aatccctatg caaacactgt atgagtccca ttgacctttc 1450

tttctcattt catttcattt ttgtct                                      1476


<210> 11
<211> 294
<212> PRT
<213> Picea glauca

<400> 11
Met Pro Leu His Phe Ser Trp Asn Pro Thr Glu Asp Pro His Thr Val
1               5                   10                  15
Arg Pro Thr Glu Ala Leu Arg Asn Gln Ser Asn Gly Arg Arg Gly Ala
            20                  25                  30
Pro Arg Leu Arg Arg Ile Lys Ser Ile Lys Tyr Trp His Arg Arg Val
        35                  40                  45
Trp Lys Leu Pro Pro Ile Ser Gly Glu Asn His Gly Glu Ala Gly Pro
    50                  55                  60
Pro Gly Ala Arg Pro Phe Gln Asp Gly Leu Tyr Gly Gly His Cys Glu
65                  70                  75                  80
Ile Gly Val Gln Phe Phe Arg Asp Ala Asp Asp Phe Cys Glu Glu His
                85                  90                  95
Pro Glu Ile Ile Leu Met Cys Ala Ser Ile Thr Leu Val Gly Gly Arg
            100                 105                 110
Ala Glu Val Ser Ala Asn Thr Ala Pro Glu Glu Glu Tyr Ala Phe Arg
        115                 120                 125
Arg Arg Pro Val Cys Glu Arg Val Ser Ala Pro Val Val Pro Ala Gly
    130                 135                 140
Phe Val Ala Arg Val Asp Val Leu Cys Thr His Pro Met Phe Gly Pro
145                 150                 155                 160
Glu Ser Ser Lys Asp Asp Leu Gly Asp Leu Pro Phe Val Tyr Asp Lys
                165                 170                 175
Val Arg Val Ser Asn Glu Gly Leu Arg Ala Lys His Cys Glu Arg Phe
            180                 185                 190
Leu Asn Ile Phe Ser Cys Glu Gly Cys Arg Met Val Glu Met Ser Cys
        195                 200                 205
Ala Glu His Asp Arg Tyr Val Ala Glu Ser Gln Phe Ile Thr His Thr
    210                 215                 220
Val Gly Arg Met Leu Gly Arg Leu Gly Leu Glu Ser Thr Pro Ile Ala

225                 230                 235                 240
Thr Lys Gly Tyr Glu Lys Leu Leu Glu Val Ala Trp Asn Ile Ala Gly
                245                 250                 255
Asp Ser Phe Asp Ile Tyr Tyr Gly Leu Phe Met Tyr Asn Val Asn Ser
            260                 265                 270
Ile Glu Gln Ile Glu Arg Leu Asp Met Ala Phe Asn Ser Leu Lys Asn
        275                 280                 285
Glu Val Ser Gly Ser Asn
    290

<210> 12
<211> 840
<212> ADN
<213> Synechocystis sp.

<220>
<221> CDS
<222> (1)..(840)

<400> 12
atg aaa att ggt gtt gtt ggt ttg ggt tta att ggg gct tcc ttg gcg      48
Met Lys Ile Gly Val Val Gly Leu Gly Leu Ile Gly Ala Ser Leu Ala
1               5                   10                  15

gga gac ttg cgt cgt cgg ggc cat tat ttg att ggg gtt tct cgg caa      96
Gly Asp Leu Arg Arg Arg Gly His Tyr Leu Ile Gly Val Ser Arg Gln
            20                  25                  30

caa agc acc tgt gaa aaa gca gtg gaa aga caa ttg gtg gat gaa gcg     144
Gln Ser Thr Cys Glu Lys Ala Val Glu Arg Gln Leu Val Asp Glu Ala
        35                  40                  45

ggt caa gat tta tct ctt ctc caa aca gca aaa ata att ttt ctt tgt     192
Gly Gln Asp Leu Ser Leu Leu Gln Thr Ala Lys Ile Ile Phe Leu Cys
    50                  55                  60

act cct ata caa tta att ttg cct acc cta gag aag ctt att ccc cat     240
Thr Pro Ile Gln Leu Ile Leu Pro Thr Leu Glu Lys Leu Ile Pro His
65                  70                  75                  80

cta tcg ccc aca gcc att gtc act gat gtg gcc tct gta aaa acg gcg     288
Leu Ser Pro Thr Ala Ile Val Thr Asp Val Ala Ser Val Lys Thr Ala
                85                  90                  95

atc gcc gag ccg gcc agt caa ctt tgg tct ggg ttc att ggt ggt cac     336
Ile Ala Glu Pro Ala Ser Gln Leu Trp Ser Gly Phe Ile Gly Gly His
            100                 105                 110

ccc atg gcc ggc aca gcg gcc cag ggc atc gac ggg gcg gaa gaa aat     384
Pro Met Ala Gly Thr Ala Ala Gln Gly Ile Asp Gly Ala Glu Glu Asn
        115                 120                 125

tta ttt gtc aac gct ccc tat gtg ctc act ccc acc gaa tat act gac     432
Leu Phe Val Asn Ala Pro Tyr Val Leu Thr Pro Thr Glu Tyr Thr Asp
    130                 135                 140

cca gag caa ttg gct tgt tta cgt tca gtg ttg gaa ccc ctg ggg gta     480
Pro Glu Gln Leu Ala Cys Leu Arg Ser Val Leu Glu Pro Leu Gly Val
145                 150                 155                 160

aaa att tac ctc tgc act ccc gca gac cat gac caa gca gta gcc tgg     528
Lys Ile Tyr Leu Cys Thr Pro Ala Asp His Asp Gln Ala Val Ala Trp

165                 170                 175

att tcc cat tta cct gta atg gtg agt gct gct tta atc caa gcc tgt     576
Ile Ser His Leu Pro Val Met Val Ser Ala Ala Leu Ile Gln Ala Cys
            180                 185                 190

gcc ggt gaa aaa gat ggg gat att ctc aaa cta gcc caa aat ttg gcc     624
Ala Gly Glu Lys Asp Gly Asp Ile Leu Lys Leu Ala Gln Asn Leu Ala
        195                 200                 205

agt tcg ggt ttt cgg gat acc agt cgg gtg gga ggc ggc aac ccg gag     672
Ser Ser Gly Phe Arg Asp Thr Ser Arg Val Gly Gly Gly Asn Pro Glu
    210                 215                 220

ttg ggc acc atg atg gcc acc tat aac caa cgg gct ttg cta aaa agt     720
Leu Gly Thr Met Met Ala Thr Tyr Asn Gln Arg Ala Leu Leu Lys Ser
225                 230                 235                 240

ttg caa gac tat cgt cag cac ctg gat cag cta att acc cta att agt     768
Leu Gln Asp Tyr Arg Gln His Leu Asp Gln Leu Ile Thr Leu Ile Ser
                245                 250                 255

aac caa caa tgg cct gaa ctc cat cgt ctt tta caa caa acc aac ggc     816
Asn Gln Gln Trp Pro Glu Leu His Arg Leu Leu Gln Gln Thr Asn Gly
            260                 265                 270

gat cgg gac aag tat gtt gaa taa                                     840
Asp Arg Asp Lys Tyr Val Glu
        275                 280

<210> 13
<211> 279
<212> PRT
<213> Synechocystis sp.

<400> 13
Met Lys Ile Gly Val Val Gly Leu Gly Leu Ile Gly Ala Ser Leu Ala
1               5                   10                  15
Gly Asp Leu Arg Arg Arg Gly His Tyr Leu Ile Gly Val Ser Arg Gln
            20                  25                  30
Gln Ser Thr Cys Glu Lys Ala Val Glu Arg Gln Leu Val Asp Glu Ala
        35                  40                  45
Gly Gln Asp Leu Ser Leu Leu Gln Thr Ala Lys Ile Ile Phe Leu Cys
    50                  55                  60
Thr Pro Ile Gln Leu Ile Leu Pro Thr Leu Glu Lys Leu Ile Pro His
65                  70                  75                  80
Leu Ser Pro Thr Ala Ile Val Thr Asp Val Ala Ser Val Lys Thr Ala
                85                  90                  95
Ile Ala Glu Pro Ala Ser Gln Leu Trp Ser Gly Phe Ile Gly Gly His
            100                 105                 110
Pro Met Ala Gly Thr Ala Ala Gln Gly Ile Asp Gly Ala Glu Glu Asn
        115                 120                 125
Leu Phe Val Asn Ala Pro Tyr Val Leu Thr Pro Thr Glu Tyr Thr Asp
    130                 135                 140
Pro Glu Gln Leu Ala Cys Leu Arg Ser Val Leu Glu Pro Leu Gly Val
145                 150                 155                 160
Lys Ile Tyr Leu Cys Thr Pro Ala Asp His Asp Gln Ala Val Ala Trp
                165                 170                 175
Ile Ser His Leu Pro Val Met Val Ser Ala Ala Leu Ile Gln Ala Cys
            180                 185                 190
Ala Gly Glu Lys Asp Gly Asp Ile Leu Lys Leu Ala Gln Asn Leu Ala
        195                 200                 205
Ser Ser Gly Phe Arg Asp Thr Ser Arg Val Gly Gly Gly Asn Pro Glu

210                 215                 220
Leu Gly Thr Met Met Ala Thr Tyr Asn Gln Arg Ala Leu Leu Lys Ser
225                 230                 235                 240
Leu Gln Asp Tyr Arg Gln His Leu Asp Gln Leu Ile Thr Leu Ile Ser
                245                 250                 255
Asn Gln Gln Trp Pro Glu Leu His Arg Leu Leu Gln Gln Thr Asn Gly
            260                 265                 270
Asp Arg Asp Lys Tyr Val Glu
        275

<210> 14
<211> 1359
<212> ADN
<213> Saccharomyces cerevisiae

<220>
<221> CDS
<222> (1)..(1359)

<400> 14
atg gta tca gag gat aag att gag caa tgg aaa gcc aca aaa gtc att      48
Met Val Ser Glu Asp Lys Ile Glu Gln Trp Lys Ala Thr Lys Val Ile
1               5                   10                  15

ggt ata att ggt ctg ggt gat atg ggc cta tta tac gct aat aaa ttt      96
Gly Ile Ile Gly Leu Gly Asp Met Gly Leu Leu Tyr Ala Asn Lys Phe
            20                  25                  30

aca gat gct gga tgg ggt gtt ata tgt tgt gat agg gaa gaa tat tat     144
Thr Asp Ala Gly Trp Gly Val Ile Cys Cys Asp Arg Glu Glu Tyr Tyr
        35                  40                  45

gat gaa ctg aaa gaa aaa tat gcc tca gct aaa ttc gaa ctg gtg aaa     192
Asp Glu Leu Lys Glu Lys Tyr Ala Ser Ala Lys Phe Glu Leu Val Lys
    50                  55                  60

aat ggt cat ttg gta tcc agg caa agc gac tat att atc tat agt gtt     240
Asn Gly His Leu Val Ser Arg Gln Ser Asp Tyr Ile Ile Tyr Ser Val
65                  70                  75                  80

gaa gca tcc aat att agt aag atc gtc gca acg tat gga cca tct tct     288
Glu Ala Ser Asn Ile Ser Lys Ile Val Ala Thr Tyr Gly Pro Ser Ser
                85                  90                  95

aag gtt gga aca att gtt ggg ggt caa acg agt tgt aag ctg ccg gaa     336
Lys Val Gly Thr Ile Val Gly Gly Gln Thr Ser Cys Lys Leu Pro Glu
            100                 105                 110

atc gag gct ttc gaa aag tat tta ccc aag gac tgc gac atc att acc     384
Ile Glu Ala Phe Glu Lys Tyr Leu Pro Lys Asp Cys Asp Ile Ile Thr
        115                 120                 125

gtg cat tcc ctt cat ggg cct aaa gtt aat act gaa ggc caa cca cta     432
Val His Ser Leu His Gly Pro Lys Val Asn Thr Glu Gly Gln Pro Leu
    130                 135                 140

gtt att atc aat cac aga tca cag tac cca gaa tct ttt gag ttc gtt     480
Val Ile Ile Asn His Arg Ser Gln Tyr Pro Glu Ser Phe Glu Phe Val
145                 150                 155                 160

aat tct gtt atg gca tgt ttg aaa agt aag caa gtt tat ttg aca tat     528
Asn Ser Val Met Ala Cys Leu Lys Ser Lys Gln Val Tyr Leu Thr Tyr

                165                 170                 175

gaa gag cat gac aag att acc gct gat aca caa gct gtg aca cat gct 576
Glu Glu His Asp Lys Ile Thr Ala Asp Thr Gln Ala Val Thr His Ala
            180                 185                 190

gct ttc tta agt atg gga tct gcg tgg gca aag ata aag att tat cct 624
Ala Phe Leu Ser Met Gly Ser Ala Trp Ala Lys Ile Lys Ile Tyr Pro
        195                 200                 205

tgg act ctg ggt gta aac aaa tgg tac ggt ggc cta gaa aat gtg aaa 672
Trp Thr Leu Gly Val Asn Lys Trp Tyr Gly Gly Leu Glu Asn Val Lys
    210                 215                 220

gtt aat ata tca cta aga atc tat tcg aac aag tgg cat gtt tac gca 720
Val Asn Ile Ser Leu Arg Ile Tyr Ser Asn Lys Trp His Val Tyr Ala
225                 230                 235                 240

gga tta gcc ata aca aac cca agt gca cat cag caa att ctt caa tat 768
Gly Leu Ala Ile Thr Asn Pro Ser Ala His Gln Gln Ile Leu Gln Tyr
                245                 250                 255

gca acc agt gca aca gaa cta ttt agt tta atg ata gat aac aaa gaa 816
Ala Thr Ser Ala Thr Glu Leu Phe Ser Leu Met Ile Asp Asn Lys Glu
            260                 265                 270

caa gaa ctt act gat aga cta tta aaa gct aag caa ttt gta ttt gga 864
Gln Glu Leu Thr Asp Arg Leu Leu Lys Ala Lys Gln Phe Val Phe Gly
        275                 280                 285

aag cat act ggt ctc tta cta ttg gat gac acg att tta gag aaa tat 912
Lys His Thr Gly Leu Leu Leu Leu Asp Asp Thr Ile Leu Glu Lys Tyr
    290                 295                 300

tcg cta tca aaa agc agc att ggt aac agc aac aat tgc aag cca gtg 960
Ser Leu Ser Lys Ser Ser Ile Gly Asn Ser Asn Asn Cys Lys Pro Val
305                 310                 315                 320

ccg aat tca cat tta tca ttg ttg gcg att gtt gat tcg tgg ttt caa 1008
Pro Asn Ser His Leu Ser Leu Leu Ala Ile Val Asp Ser Trp Phe Gln
                325                 330                 335

ctt ggt att gat cca tat gat cat atg att tgt tcg acg cca tta ttc 1056
Leu Gly Ile Asp Pro Tyr Asp His Met Ile Cys Ser Thr Pro Leu Phe
            340                 345                 350

aga ata ttc ctg ggt gtg tcc gaa tat ctt ttt tta aaa cct ggc tta 1104
Arg Ile Phe Leu Gly Val Ser Glu Tyr Leu Phe Leu Lys Pro Gly Leu
        355                 360                 365

tta gaa cag aca att gat gca gct atc cat gat aaa tca ttc ata aaa 1152
Leu Glu Gln Thr Ile Asp Ala Ala Ile His Asp Lys Ser Phe Ile Lys
    370                 375                 380

gat gat tta gaa ttt gtt att tcg gct aga gaa tgg agc tcg gtt gtt 1200
Asp Asp Leu Glu Phe Val Ile Ser Ala Arg Glu Trp Ser Ser Val Val
385                 390                 395                 400

tct ttt gcc aat ttt gat ata tac aaa aag caa ttt cag agt gtt caa 1248
Ser Phe Ala Asn Phe Asp Ile Tyr Lys Lys Gln Phe Gln Ser Val Gln
                405                 410                 415

aag ttc ttt gag cca atg ctt cca gag gct aat ctc att ggc aac gag 1296
Lys Phe Phe Glu Pro Met Leu Pro Glu Ala Asn Leu Ile Gly Asn Glu

420                 425                 430

atg ata aaa acc att ctg agt cat tct agt gac cgt tcg gcc gct gaa     1344
Met Ile Lys Thr Ile Leu Ser His Ser Ser Asp Arg Ser Ala Ala Glu
        435                 440                 445

aaa aga aat aca taa                                                 1359
Lys Arg Asn Thr
    450

<210> 15
<211> 452
<212> PRT
<213> Saccharomyces cerevisiae

<400> 15
Met Val Ser Glu Asp Lys Ile Glu Gln Trp Lys Ala Thr Lys Val Ile
1               5                   10                  15
Gly Ile Ile Gly Leu Gly Asp Met Gly Leu Leu Tyr Ala Asn Lys Phe
            20                  25                  30
Thr Asp Ala Gly Trp Gly Val Ile Cys Cys Asp Arg Glu Glu Tyr Tyr
        35                  40                  45
Asp Glu Leu Lys Glu Lys Tyr Ala Ser Ala Lys Phe Glu Leu Val Lys
    50                  55                  60
Asn Gly His Leu Val Ser Arg Gln Ser Asp Tyr Ile Ile Tyr Ser Val
65                  70                  75                  80
Glu Ala Ser Asn Ile Ser Lys Ile Val Ala Thr Tyr Gly Pro Ser Ser
                85                  90                  95
Lys Val Gly Thr Ile Val Gly Gly Gln Thr Ser Cys Lys Leu Pro Glu
            100                 105                 110
Ile Glu Ala Phe Glu Lys Tyr Leu Pro Lys Asp Cys Asp Ile Ile Thr
        115                 120                 125
Val His Ser Leu His Gly Pro Lys Val Asn Thr Glu Gly Gln Pro Leu
    130                 135                 140
Val Ile Ile Asn His Arg Ser Gln Tyr Pro Glu Ser Phe Glu Phe Val
145                 150                 155                 160
Asn Ser Val Met Ala Cys Leu Lys Ser Lys Gln Val Tyr Leu Thr Tyr
                165                 170                 175
Glu Glu His Asp Lys Ile Thr Ala Asp Thr Gln Ala Val Thr His Ala
            180                 185                 190
Ala Phe Leu Ser Met Gly Ser Ala Trp Ala Lys Ile Lys Ile Tyr Pro
        195                 200                 205
Trp Thr Leu Gly Val Asn Lys Trp Tyr Gly Gly Leu Glu Asn Val Lys
    210                 215                 220
Val Asn Ile Ser Leu Arg Ile Tyr Ser Asn Lys Trp His Val Tyr Ala
225                 230                 235                 240
Gly Leu Ala Ile Thr Asn Pro Ser Ala His Gln Gln Ile Leu Gln Tyr
                245                 250                 255
Ala Thr Ser Ala Thr Glu Leu Phe Ser Leu Met Ile Asp Asn Lys Glu
            260                 265                 270
Gln Glu Leu Thr Asp Arg Leu Leu Lys Ala Lys Gln Phe Val Phe Gly
        275                 280                 285
Lys His Thr Gly Leu Leu Leu Leu Asp Asp Thr Ile Leu Glu Lys Tyr
    290                 295                 300
Ser Leu Ser Lys Ser Ser Ile Gly Asn Ser Asn Asn Cys Lys Pro Val
305                 310                 315                 320
Pro Asn Ser His Leu Ser Leu Leu Ala Ile Val Asp Ser Trp Phe Gln
                325                 330                 335
Leu Gly Ile Asp Pro Tyr Asp His Met Ile Cys Ser Thr Pro Leu Phe
            340                 345                 350
Arg Ile Phe Leu Gly Val Ser Glu Tyr Leu Phe Leu Lys Pro Gly Leu
        355                 360                 365
Leu Glu Gln Thr Ile Asp Ala Ala Ile His Asp Lys Ser Phe Ile Lys

        370                 375                 380
Asp Asp Leu Glu Phe Val Ile Ser Ala Arg Glu Trp Ser Ser Val Val
385                 390                 395                 400
Ser Phe Ala Asn Phe Asp Ile Tyr Lys Lys Gln Phe Gln Ser Val Gln
                405                 410                 415
Lys Phe Phe Glu Pro Met Leu Pro Glu Ala Asn Leu Ile Gly Asn Glu
            420                 425                 430
Met Ile Lys Thr Ile Leu Ser His Ser Ser Asp Arg Ser Ala Ala Glu
        435                 440                 445
Lys Arg Asn Thr
    450




<210> 16
<211> 1116
<212> ADN
<213> Bacillus subtilis

<220>
<221> CDS
<222> (1)..(1116)

<400> 16
atg aat caa atg aaa gat aca ata ttg ctc gcc ggt ctc gga ttg ata      48
Met Asn Gln Met Lys Asp Thr Ile Leu Leu Ala Gly Leu Gly Leu Ile
1               5                   10                  15

ggc ggt tcg att gcc cta gcc atc aaa aaa aat cat ccc ggc aaa cgg      96
Gly Gly Ser Ile Ala Leu Ala Ile Lys Lys Asn His Pro Gly Lys Arg
            20                  25                  30

att atc gga atc gac atc tct gat gaa cag gcg gta gcg gca tta aaa      144
Ile Ile Gly Ile Asp Ile Ser Asp Glu Gln Ala Val Ala Ala Leu Lys
        35                  40                  45

tta ggc gtg ata gac gat cgt gct gat tcg ttt att agc ggt gtg aaa      192
Leu Gly Val Ile Asp Asp Arg Ala Asp Ser Phe Ile Ser Gly Val Lys
    50                  55                  60

gag gca gct aca gta atc att gcg aca cct gtt gaa caa aca ctg gtt      240
Glu Ala Ala Thr Val Ile Ile Ala Thr Pro Val Glu Gln Thr Leu Val
65                  70                  75                  80

atg ctt gaa gag ctg gct cat tca gga att gaa cat gag ctt ttg att      288
Met Leu Glu Glu Leu Ala His Ser Gly Ile Glu His Glu Leu Leu Ile
                85                  90                  95

acg gat gta gga agc aca aag caa aaa gtg gtt gat tac gct gat caa      336
Thr Asp Val Gly Ser Thr Lys Gln Lys Val Val Asp Tyr Ala Asp Gln
            100                 105                 110

gtg ctg cct agc cgc tat caa ttt gtc gga ggg cat ccg atg gcg ggt      384
Val Leu Pro Ser Arg Tyr Gln Phe Val Gly Gly His Pro Met Ala Gly
        115                 120                 125

tca cat aaa tca gga gtg gcc gct gcg aag gag ttc ctg ttt gaa aat      432
Ser His Lys Ser Gly Val Ala Ala Ala Lys Glu Phe Leu Phe Glu Asn
    130                 135                 140

gca ttt tat att tta acg cca ggc cag aaa acg gac aaa caa gct gtg      480
Ala Phe Tyr Ile Leu Thr Pro Gly Gln Lys Thr Asp Lys Gln Ala Val
145                 150                 155                 160

gaa cag tta aaa aac ctg ctg aag ggg acg aat gcc cat ttt gtg gaa    528
Glu Gln Leu Lys Asn Leu Leu Lys Gly Thr Asn Ala His Phe Val Glu
                165                 170                 175

atg tcg cca gag gag cat gat ggc gtt aca agc gta atc agt cat ttt    576
Met Ser Pro Glu Glu His Asp Gly Val Thr Ser Val Ile Ser His Phe
            180                 185                 190

ccg cat att gta gca gct agc ctt gtt cac caa acc cat cat tcg gaa    624
Pro His Ile Val Ala Ala Ser Leu Val His Gln Thr His His Ser Glu
        195                 200                 205

aac ctg tat ccg ctt gtt aag cgt ttt gct gcc ggc ggg ttc aga gat    672
Asn Leu Tyr Pro Leu Val Lys Arg Phe Ala Ala Gly Gly Phe Arg Asp
    210                 215                 220

att aca agg att gca tca agc agc ccg gca atg tgg cgg gat att tta    720
Ile Thr Arg Ile Ala Ser Ser Ser Pro Ala Met Trp Arg Asp Ile Leu
225                 230                 235                 240

tta cat aat aaa gat aaa atc tta gac cgt ttt gat gag tgg att cgt    768
Leu His Asn Lys Asp Lys Ile Leu Asp Arg Phe Asp Glu Trp Ile Arg
                245                 250                 255

gaa att gac aag atc cgt aca tat gta gaa caa gaa gat gcg gaa aat    816
Glu Ile Asp Lys Ile Arg Thr Tyr Val Glu Gln Glu Asp Ala Glu Asn
            260                 265                 270

cta ttt cgt tat ttt aaa aca gcc aag gat tat cgc gac ggg ctg ccg    864
Leu Phe Arg Tyr Phe Lys Thr Ala Lys Asp Tyr Arg Asp Gly Leu Pro
        275                 280                 285

ctt cgg cag aag gga gcg ata cct gca ttt tat gat tta tat gtg gat    912
Leu Arg Gln Lys Gly Ala Ile Pro Ala Phe Tyr Asp Leu Tyr Val Asp
    290                 295                 300

gta ccc gat cat ccg ggt gta ata tcc gag ata aca gcg atc tta gct    960
Val Pro Asp His Pro Gly Val Ile Ser Glu Ile Thr Ala Ile Leu Ala
305                 310                 315                 320

gcg gag cgc atc agt atc acg aat atc cgc att atc gaa aca cga gag    1008
Ala Glu Arg Ile Ser Ile Thr Asn Ile Arg Ile Ile Glu Thr Arg Glu
                325                 330                 335

gat att aac ggg att tta agg atc agt ttt cag tct gat gac gac cgc    1056
Asp Ile Asn Gly Ile Leu Arg Ile Ser Phe Gln Ser Asp Asp Asp Arg
            340                 345                 350

aaa agg gca gaa caa tgc att gaa gcc cgg gcg gaa tat gaa act ttt    1104
Lys Arg Ala Glu Gln Cys Ile Glu Ala Arg Ala Glu Tyr Glu Thr Phe
        355                 360                 365

tat gct gat tga                                                    1116
Tyr Ala Asp
    370

<210> 17
<211> 371
<212> PRT
<213> Bacillus subtilis

<400> 17
Met Asn Gln Met Lys Asp Thr Ile Leu Leu Ala Gly Leu Gly Leu Ile

1               5                   10                  15
Gly Gly Ser Ile Ala Leu Ala Ile Lys Lys Asn His Pro Gly Lys Arg
            20                  25                  30
Ile Ile Gly Ile Asp Ile Ser Asp Glu Gln Ala Val Ala Ala Leu Lys
        35                  40                  45
Leu Gly Val Ile Asp Asp Arg Ala Asp Ser Phe Ile Ser Gly Val Lys
    50                  55                  60
Glu Ala Ala Thr Val Ile Ile Ala Thr Pro Val Glu Gln Thr Leu Val
65                  70                  75                  80
Met Leu Glu Glu Leu Ala His Ser Gly Ile Glu His Glu Leu Leu Ile
                85                  90                  95
Thr Asp Val Gly Ser Thr Lys Gln Lys Val Val Asp Tyr Ala Asp Gln
            100                 105                 110
Val Leu Pro Ser Arg Tyr Gln Phe Val Gly Gly His Pro Met Ala Gly
        115                 120                 125
Ser His Lys Ser Gly Val Ala Ala Ala Lys Glu Phe Leu Phe Glu Asn
    130                 135                 140
Ala Phe Tyr Ile Leu Thr Pro Gly Gln Lys Thr Asp Lys Gln Ala Val
145                 150                 155                 160
Glu Gln Leu Lys Asn Leu Leu Lys Gly Thr Asn Ala His Phe Val Glu
                165                 170                 175
Met Ser Pro Glu Glu His Asp Gly Val Thr Ser Val Ile Ser His Phe
            180                 185                 190
Pro His Ile Val Ala Ala Ser Leu Val His Gln Thr His His Ser Glu
        195                 200                 205
Asn Leu Tyr Pro Leu Val Lys Arg Phe Ala Ala Gly Gly Phe Arg Asp
    210                 215                 220
Ile Thr Arg Ile Ala Ser Ser Ser Pro Ala Met Trp Arg Asp Ile Leu
225                 230                 235                 240
Leu His Asn Lys Asp Lys Ile Leu Asp Arg Phe Asp Glu Trp Ile Arg
                245                 250                 255
Glu Ile Asp Lys Ile Arg Thr Tyr Val Glu Gln Glu Asp Ala Glu Asn
            260                 265                 270
Leu Phe Arg Tyr Phe Lys Thr Ala Lys Asp Tyr Arg Asp Gly Leu Pro
        275                 280                 285
Leu Arg Gln Lys Gly Ala Ile Pro Ala Phe Tyr Asp Leu Tyr Val Asp
    290                 295                 300
Val Pro Asp His Pro Gly Val Ile Ser Glu Ile Thr Ala Ile Leu Ala
305                 310                 315                 320
Ala Glu Arg Ile Ser Ile Thr Asn Ile Arg Ile Ile Glu Thr Arg Glu
                325                 330                 335
Asp Ile Asn Gly Ile Leu Arg Ile Ser Phe Gln Ser Asp Asp Asp Arg
            340                 345                 350
Lys Arg Ala Glu Gln Cys Ile Glu Ala Arg Ala Glu Tyr Glu Thr Phe
        355                 360                 365
Tyr Ala Asp
    370

<210> 18
<211> 1122
<212> ADN
<213> Escherichia coli

<220>
<221> CDS
<222> (1)..(1122)

<400> 18
atg gtt gct gaa ttg acc gca tta cgc gat caa att gat gaa gtc gat      48
Met Val Ala Glu Leu Thr Ala Leu Arg Asp Gln Ile Asp Glu Val Asp
1               5                   10                  15

aaa gcg ctg ctg aat tta tta gcg aag cgt ctg gaa ctg gtt gct gaa    96
Lys Ala Leu Leu Asn Leu Leu Ala Lys Arg Leu Glu Leu Val Ala Glu
            20                  25                  30

gtg ggc gag gtg aaa agc cgc ttt gga ctg cct att tat gtt ccg gag    144
Val Gly Glu Val Lys Ser Arg Phe Gly Leu Pro Ile Tyr Val Pro Glu
        35                  40                  45

cgc gag gca tct atg ttg gcc tcg cgt cgt gca gag gcg gaa gct ctg    192
Arg Glu Ala Ser Met Leu Ala Ser Arg Arg Ala Glu Ala Glu Ala Leu
    50                  55                  60

ggt gta ccg cca gat ctg att gag gat gtt ttg cgt cgg gtg atg cgt    240
Gly Val Pro Pro Asp Leu Ile Glu Asp Val Leu Arg Arg Val Met Arg
65                  70                  75                  80

gaa tct tac tcc agt gaa aac gac aaa gga ttt aaa aca ctt tgt ccg    288
Glu Ser Tyr Ser Ser Glu Asn Asp Lys Gly Phe Lys Thr Leu Cys Pro
                85                  90                  95

tca ctg cgt ccg gtg gtt atc gtc ggc ggt ggc ggt cag atg gga cgc    336
Ser Leu Arg Pro Val Val Ile Val Gly Gly Gly Gly Gln Met Gly Arg
            100                 105                 110

ctg ttc gag aag atg ctg acc ctc tcg ggt tat cag gtg cgg att ctg    384
Leu Phe Glu Lys Met Leu Thr Leu Ser Gly Tyr Gln Val Arg Ile Leu
        115                 120                 125

gag caa cat gac tgg gat cga gcg gct gat att gtt gcc gat gcc gga    432
Glu Gln His Asp Trp Asp Arg Ala Ala Asp Ile Val Ala Asp Ala Gly
    130                 135                 140

atg gtg att gtt agt gtg cca atc cac gtt act gag caa gtt att ggc    480
Met Val Ile Val Ser Val Pro Ile His Val Thr Glu Gln Val Ile Gly
145                 150                 155                 160

aaa tta ccg cct tta ccg aaa gat tgt att ctg gtc gat ctg gca tca    528
Lys Leu Pro Pro Leu Pro Lys Asp Cys Ile Leu Val Asp Leu Ala Ser
                165                 170                 175

gtg aaa aat ggg cca tta cag gcc atg ctg gtg gcg cat gat ggt ccg    576
Val Lys Asn Gly Pro Leu Gln Ala Met Leu Val Ala His Asp Gly Pro
            180                 185                 190

gtg ctg ggg cta cac ccg atg ttc ggt ccg gac agc ggt agc ctg gca    624
Val Leu Gly Leu His Pro Met Phe Gly Pro Asp Ser Gly Ser Leu Ala
        195                 200                 205

aag caa gtt gtg gtc tgg tgt gat gga cgt aaa ccg gaa gca tac caa    672
Lys Gln Val Val Val Trp Cys Asp Gly Arg Lys Pro Glu Ala Tyr Gln
    210                 215                 220

tgg ttt ctg gag caa att cag gtc tgg ggc gct cgg ctg cat cgt att    720
Trp Phe Leu Glu Gln Ile Gln Val Trp Gly Ala Arg Leu His Arg Ile
225                 230                 235                 240

agc gcc gtc gag cac gat cag aat atg gcg ttt att cag gca ctg cgc    768
Ser Ala Val Glu His Asp Gln Asn Met Ala Phe Ile Gln Ala Leu Arg
                245                 250                 255

cac ttt gct act ttt gct tac ggg ctg cac ctg gca gaa gaa aat gtt    816
His Phe Ala Thr Phe Ala Tyr Gly Leu His Leu Ala Glu Glu Asn Val
            260                 265                 270

cag ctt gag caa ctt ctg gcg ctc tct tcg ccg att tac cgc ctt gag     864
Gln Leu Glu Gln Leu Leu Ala Leu Ser Ser Pro Ile Tyr Arg Leu Glu
        275                 280                 285

ctg gcg atg gtc ggg cga ctg ttt gct cag gat ccg cag ctt tat gcc     912
Leu Ala Met Val Gly Arg Leu Phe Ala Gln Asp Pro Gln Leu Tyr Ala
    290                 295                 300

gac atc att atg tcg tca gag cgt aat ctg gcg tta atc aaa cgt tac     960
Asp Ile Ile Met Ser Ser Glu Arg Asn Leu Ala Leu Ile Lys Arg Tyr
305                 310                 315                 320

tat aag cgt ttc ggc gag gcg att gag ttg ctg gag cag ggc gat aag     1008
Tyr Lys Arg Phe Gly Glu Ala Ile Glu Leu Leu Glu Gln Gly Asp Lys
                325                 330                 335

cag gcg ttt att gac agt ttc cgc aag gtg gag cac tgg ttc ggc gat     1056
Gln Ala Phe Ile Asp Ser Phe Arg Lys Val Glu His Trp Phe Gly Asp
            340                 345                 350

tac gca cag cgt ttt cag agt gaa agc cgc gtg tta ttg cgt cag gcg     1104
Tyr Ala Gln Arg Phe Gln Ser Glu Ser Arg Val Leu Leu Arg Gln Ala
        355                 360                 365

aat gac aat cgc cag taa                                             1122
Asn Asp Asn Arg Gln
    370


<210> 19
<211> 373
<212> PRT
<213> Escherichia coli

<400> 19
Met Val Ala Glu Leu Thr Ala Leu Arg Asp Gln Ile Asp Glu Val Asp
  1               5                  10                  15
Lys Ala Leu Leu Asn Leu Leu Ala Lys Arg Leu Glu Leu Val Ala Glu
             20                  25                  30
Val Gly Glu Val Lys Ser Arg Phe Gly Leu Pro Ile Tyr Val Pro Glu
         35                  40                  45
Arg Glu Ala Ser Met Leu Ala Ser Arg Arg Ala Glu Ala Glu Ala Leu
     50                  55                  60
Gly Val Pro Pro Asp Leu Ile Glu Asp Val Leu Arg Arg Val Met Arg
 65                  70                  75                  80
Glu Ser Tyr Ser Ser Glu Asn Asp Lys Gly Phe Lys Thr Leu Cys Pro
                 85                  90                  95
Ser Leu Arg Pro Val Val Ile Val Gly Gly Gly Gly Gln Met Gly Arg
            100                 105                 110
Leu Phe Glu Lys Met Leu Thr Leu Ser Gly Tyr Gln Val Arg Ile Leu
        115                 120                 125
Glu Gln His Asp Trp Asp Arg Ala Ala Asp Ile Val Ala Asp Ala Gly
    130                 135                 140
Met Val Ile Val Ser Val Pro Ile His Val Thr Glu Gln Val Ile Gly
145                 150                 155                 160
Lys Leu Pro Pro Leu Pro Lys Asp Cys Ile Leu Val Asp Leu Ala Ser
                165                 170                 175
Val Lys Asn Gly Pro Leu Gln Ala Met Leu Val Ala His Asp Gly Pro
            180                 185                 190
Val Leu Gly Leu His Pro Met Phe Gly Pro Asp Ser Gly Ser Leu Ala
        195                 200                 205
Lys Gln Val Val Val Trp Cys Asp Gly Arg Lys Pro Glu Ala Tyr Gln
    210                 215                 220
Trp Phe Leu Glu Gln Ile Gln Val Trp Gly Ala Arg Leu His Arg Ile

225                 230                     235                     240
Ser Ala Val Glu His Asp Gln Asn Met Ala Phe Ile Gln Ala Leu Arg
                245                     250                     255
His Phe Ala Thr Phe Ala Tyr Gly Leu His Leu Ala Glu Glu Asn Val
            260                     265                     270
Gln Leu Glu Gln Leu Leu Ala Leu Ser Ser Pro Ile Tyr Arg Leu Glu
        275                     280                     285
Leu Ala Met Val Gly Arg Leu Phe Ala Gln Asp Pro Gln Leu Tyr Ala
    290                     295                     300
Asp Ile Ile Met Ser Ser Glu Arg Asn Leu Ala Leu Ile Lys Arg Tyr
305                     310                     315                     320
Tyr Lys Arg Phe Gly Glu Ala Ile Glu Leu Leu Glu Gln Gly Asp Lys
                325                     330                     335
Gln Ala Phe Ile Asp Ser Phe Arg Lys Val Glu His Trp Phe Gly Asp
            340                     345                     350
Tyr Ala Gln Arg Phe Gln Ser Glu Ser Arg Val Leu Leu Arg Gln Ala
        355                     360                     365
Asn Asp Asn Arg Gln
    370

<210> 20
<211> 1152
<212> ADN
<213> Erwinia herbicola

<220>
<221> CDS
<222> (1)..(1119)

<400> 20
atg gtg gct gaa ctg acc gcg tta cgc gat caa att gac agt gta gat    48
Met Val Ala Glu Leu Thr Ala Leu Arg Asp Gln Ile Asp Ser Val Asp
  1               5                  10                  15

aaa gcg ctg ctg gat ctg ctg gct aag cga ctg gaa ctg gtg gcc gag    96
Lys Ala Leu Leu Asp Leu Leu Ala Lys Arg Leu Glu Leu Val Ala Glu
             20                  25                  30

gta ggt gag gtg aag agc cgt tac ggc ctg cct atc tat gtg cct gag    144
Val Gly Glu Val Lys Ser Arg Tyr Gly Leu Pro Ile Tyr Val Pro Glu
         35                  40                  45

cgt gag gcg tcg atg ctg gct tcg cgt cgc aaa gag gcc gaa gcg ctc    192
Arg Glu Ala Ser Met Leu Ala Ser Arg Arg Lys Glu Ala Glu Ala Leu
     50                  55                  60

ggc gta cca ccg gat ctg att gag gat gtg ctg cgt cgc gtg atg cgg    240
Gly Val Pro Pro Asp Leu Ile Glu Asp Val Leu Arg Arg Val Met Arg
 65                  70                  75                  80

gaa tcc tat acc agc gag aat gat aaa ggc ttt aaa acc ctc tgt cct    288
Glu Ser Tyr Thr Ser Glu Asn Asp Lys Gly Phe Lys Thr Leu Cys Pro
                 85                  90                  95

gaa ctg cgc ccg gtg gtg att gtc ggt ggt aag ggc cag atg ggc cgg    336
Glu Leu Arg Pro Val Val Ile Val Gly Gly Lys Gly Gln Met Gly Arg
            100                 105                 110

ctg ttt gaa aaa atg ctc ggg cta tca ggc tac acg gtt aaa acg ctg    384
Leu Phe Glu Lys Met Leu Gly Leu Ser Gly Tyr Thr Val Lys Thr Leu
        115                 120                 125

gat aaa gag gac tgg cct cag gct gag act ctg ctc agc gat gcc gga     432
Asp Lys Glu Asp Trp Pro Gln Ala Glu Thr Leu Leu Ser Asp Ala Gly
    130                 135                 140

atg gtg atc att agc gtg ccg att cac ctg acc gag cag gtg att gcc     480
Met Val Ile Ile Ser Val Pro Ile His Leu Thr Glu Gln Val Ile Ala
145                 150                 155                 160

caa ctg cca cca ctg ccg gaa gat tgt att ctg gtc gat ctg gcg tca     528
Gln Leu Pro Pro Leu Pro Glu Asp Cys Ile Leu Val Asp Leu Ala Ser
                165                 170                 175

gtc aaa aac cgg cct ctg cag gca atg ctg gct gcc cat aac ggg cct     576
Val Lys Asn Arg Pro Leu Gln Ala Met Leu Ala Ala His Asn Gly Pro
            180                 185                 190

gta ctg ggt ctg cat ccg atg ttt ggc ccg gac agc ggc agc ctg gca     624
Val Leu Gly Leu His Pro Met Phe Gly Pro Asp Ser Gly Ser Leu Ala
        195                 200                 205

aaa cag gtg gtg gtc tgg tgt gat gga aga caa ccg gaa gcg tat cag     672
Lys Gln Val Val Val Trp Cys Asp Gly Arg Gln Pro Glu Ala Tyr Gln
    210                 215                 220

tgg ttc ctg gag cag att cag gtc tgg ggt gcg cgt ctg cat cgt atc     720
Trp Phe Leu Glu Gln Ile Gln Val Trp Gly Ala Arg Leu His Arg Ile
225                 230                 235                 240

agc gct gtt gag cat gac cag aac atg gca ttc att cag gcg ctg cgt     768
Ser Ala Val Glu His Asp Gln Asn Met Ala Phe Ile Gln Ala Leu Arg
                245                 250                 255

cac ttt gct acc ttc gct tat ggt ctg cat tta gcc gaa gag aac gtc     816
His Phe Ala Thr Phe Ala Tyr Gly Leu His Leu Ala Glu Glu Asn Val
            260                 265                 270

aat ctg gat cag ctg ctg gcg ctc tcg tcg ccc att tac cgg ctt gaa     864
Asn Leu Asp Gln Leu Leu Ala Leu Ser Ser Pro Ile Tyr Arg Leu Glu
        275                 280                 285

ctg gcg atg gtg ggg cgg ttg ttc gct cag gat ccg caa ctc tat gcg     912
Leu Ala Met Val Gly Arg Leu Phe Ala Gln Asp Pro Gln Leu Tyr Ala
    290                 295                 300

gat atc atc atg tct tca gag agt aat ctg gcg ctg ata aaa cgc tat     960
Asp Ile Ile Met Ser Ser Glu Ser Asn Leu Ala Leu Ile Lys Arg Tyr
305                 310                 315                 320

tac cag cgg ttt ggt gaa gcg att gcg ctg ctg gag cag ggc gac aag     1008
Tyr Gln Arg Phe Gly Glu Ala Ile Ala Leu Leu Glu Gln Gly Asp Lys
                325                 330                 335

cag gcg ttt atc gcc agc ttt aac cgg gtt gaa cag tgg ttt ggc gat     1056
Gln Ala Phe Ile Ala Ser Phe Asn Arg Val Glu Gln Trp Phe Gly Asp
            340                 345                 350

cac gca aaa cgc ttc ctg gtc gaa agc cga agc ctg ttg cga tcg gcc     1104
His Ala Lys Arg Phe Leu Val Glu Ser Arg Ser Leu Leu Arg Ser Ala
        355                 360                 365

aat gac agc cgc cca taaaaaaaag gcatccagtt ggatgccttt ttt            1152
Asn Asp Ser Arg Pro
    370

<210> 21
<211> 373
<212> PRT
<213> Erwinia herbicola

<400> 21
Met Val Ala Glu Leu Thr Ala Leu Arg Asp Gln Ile Asp Ser Val Asp
1               5                   10                  15

Lys Ala Leu Leu Asp Leu Leu Ala Lys Arg Leu Glu Leu Val Ala Glu
            20                  25                  30

Val Gly Glu Val Lys Ser Arg Tyr Gly Leu Pro Ile Tyr Val Pro Glu
        35                  40                  45

Arg Glu Ala Ser Met Leu Ala Ser Arg Arg Lys Glu Ala Glu Ala Leu
    50                  55                  60

Gly Val Pro Pro Asp Leu Ile Glu Asp Val Leu Arg Arg Val Met Arg
65                  70                  75                  80

Glu Ser Tyr Thr Ser Glu Asn Asp Lys Gly Phe Lys Thr Leu Cys Pro
                85                  90                  95

Glu Leu Arg Pro Val Val Ile Val Gly Gly Lys Gly Gln Met Gly Arg
            100                 105                 110

Leu Phe Glu Lys Met Leu Gly Leu Ser Gly Tyr Thr Val Lys Thr Leu
        115                 120                 125

Asp Lys Glu Asp Trp Pro Gln Ala Glu Thr Leu Leu Ser Asp Ala Gly
    130                 135                 140

Met Val Ile Ile Ser Val Pro Ile His Leu Thr Glu Gln Val Ile Ala
145                 150                 155                 160

Gln Leu Pro Pro Leu Pro Glu Asp Cys Ile Leu Val Asp Leu Ala Ser
                165                 170                 175

Val Lys Asn Arg Pro Leu Gln Ala Met Leu Ala Ala His Asn Gly Pro
            180                 185                 190

Val Leu Gly Leu His Pro Met Phe Gly Pro Asp Ser Gly Ser Leu Ala
        195                 200                 205

Lys Gln Val Val Val Trp Cys Asp Gly Arg Gln Pro Glu Ala Tyr Gln
    210                 215                 220

Trp Phe Leu Glu Gln Ile Gln Val Trp Gly Ala Arg Leu His Arg Ile
225                 230                 235                 240

Ser Ala Val Glu His Asp Gln Asn Met Ala Phe Ile Gln Ala Leu Arg
                245                 250                 255

His Phe Ala Thr Phe Ala Tyr Gly Leu His Leu Ala Glu Glu Asn Val
            260                 265                 270

Asn Leu Asp Gln Leu Leu Ala Leu Ser Ser Pro Ile Tyr Arg Leu Glu
        275                 280                 285

Leu Ala Met Val Gly Arg Leu Phe Ala Gln Asp Pro Gln Leu Tyr Ala
    290                 295                 300

Asp Ile Ile Met Ser Ser Glu Ser Asn Leu Ala Leu Ile Lys Arg Tyr
305                 310                 315                 320

Tyr Gln Arg Phe Gly Glu Ala Ile Ala Leu Leu Glu Gln Gly Asp Lys
                325                 330                 335

Gln Ala Phe Ile Ala Ser Phe Asn Arg Val Glu Gln Trp Phe Gly Asp
            340                 345                 350

His Ala Lys Arg Phe Leu Val Glu Ser Arg Ser Leu Leu Arg Ser Ala
        355                 360                 365

Asn Asp Ser Arg Pro
    370

<210> 22
<211> 1129
<212> ADN
<213> Lycopersicon esculentum

<400> 22
atgttttccc tttcatctat acaatctaac aatattcaat ctcaatcatc ttcgtcgcta 60
ctcttcaatc atcatcacca gcattcaact atttcaactc ggtttcatca ccaccgccta 120
ctcttccctc tccgtgccca aaatagcgac ttaactacag ccaccaccaa taacaactat 180
gtcgatcttg atgacaatct aaccagactt gataaatttt caaaatcatt aagtatttcg 240
aatatcgaag aaaatacatc attaaatccc ctcttatgtt ccaataacaa gctcaaaata 300
gctatcatag gctttggaaa ctttggacaa tttattgcca aatcctttat caaacaaggc 360
catgttgtat tagctcattc acgtagtgat tattccctca tagcacaatc ccttaatgtc 420
cacttctttc aagatcctaa tgacttatgt gaacaacatc ctgacgttat tttactttgc 480
acatccatca attcactcga aaacgtcatt cgttcccttc ccatccaaaa gcttaaacgt 540
aacacacttt tcgtagacgt attatcagtc aaagaattcc cgaaaaacat ttttcttcaa 600
tcactaccaa aagaatttga tattttgtgt actcatccta tgtttggtcc aacaagtggt 660
aaagacaatt ggaaaggact accatttatg tatgacaaag ttagaattgg acaagaagag 720
tcaagaatta aaagagtcaa caattttatc aacatttttg taaaagaagg ttgtagaatg 780
gttgaaatga gttgtagtga acatgacaag tatgctgctg gatcacaatt tattacacat 840
actattggaa gaatgttaca aagacttggg acacaaacaa ctcctataaa cacaaaagga 900
tatgaaagtt tgttgaattt gatggagaat acaactagtg atagttttga tttgtattgt 960
ggtttgctta tgtataacaa taattcaatg gaggtgttag agaaactaga tgcagcattg 1020
gatagtttga aaagggaatt atttggacaa gttcttcaaa agttggagaa aagagtggaa 1080
aagggaagta agttagcttt acctactcct gattttagta agaaaattg          1129

<210> 23
<211> 32
<212> ADN
<213> Séquence artificielle

<220>
<223> Description de la séquence artificielle:
      Oligonucléotide P1

<400> 23
tctccatatg atctttcaat ctcattctca tc                               32

<210> 24
<211> 32
<212> ADN
<213> Séquence artificielle

<220>
<223> Description de la séquence artificielle:

Oligonucléotide P2

<400> 24
ctaactaact aactacatac ctcatcatat cc                                    32


<210> 25
<211> 26
<212> ADN
<213> Séquence artificielle

<220>
<223> Description de la séquence artificielle:
      Oligonucléotide P3

<400> 25
cctctctttc catatgctcc cttctc                                           26


<210> 26
<211> 33
<212> ADN
<213> Séquence artificielle

<220>
<223> Description de la séquence artificielle:
      Oligonucléotide P4

<400> 26
ccgccagcca cctccatatg accgacacca tcc                                   33


<210> 27
<211> 25
<212> ADN
<213> Séquence artificielle

<220>
<223> Description de la séquence artificielle:
      Oligonucléotide P5

<400> 27
cgccacccct catatgcgta tcgcc                                            25


<210> 28
<211> 34
<212> ADN
<213> Séquence artificielle

<220>
<223> Description de la séquence artificielle:
      Oligonucléotide P6

<400> 28
gatgcatctt tgcatatgat gaggtcagaa gatg                                  34


<210> 29
<211> 35
<212> ADN
<213> Séquence artificielle

<220>

<223> Description de la séquence artificielle:
      Oligonucléotide P7

<400> 29
cagtataatt agtagtcaag gatcctgact gagag                                35


<210> 30
<211> 31
<212> ADN
<213> Séquence artificielle

<220>
<223> Description de la séquence artificielle:
      Oligonucléotide P8

<400> 30
gctaaaactc ttctccttca atacttacct g                                    31

## Description

La présente invention a pour objet de nouvelles enzymes possédant une activité arogénate déhydrogénase, en particulier des enzymes arogénate déhydrogénase de plantes, ainsi que les gènes codant ces enzymes. Les enzymes arogénate déhydrogénase selon l'invention catalysent la dernière étape de la voie métabolique de biosynthèse de la tyrosine, et constituent, à ce titre, des cibles potentielles d'herbicides. La présente invention concerne donc également un procédé d'identification de composés herbicides ayant pour cible ces enzymes, lesdits composés herbicides empêchant la biosynthèse de tyrosine en se fixant sur lesdites enzymes. L'invention a également pour objet des plantes transgénique tolérantes à des composés herbicides ayant pour cible une enzyme impliquée dans la voie de biosynthèse de la tyrosine, en particulier une enzyme impliquée dans la transformation du préphénate en L-tyrosine, en particulier une enzyme arogénate déhydrogénase. Ces plantes deviennent tolérantes par expression dans leurs tissus d'une enzyme préphénate dehydrogénase, cette enzyme étant insensible aux dits composés herbicides et permettant à la plante de synthétiser la tyrosine malgré un traitement par lesdits composés herbicides.

La voie de biosynthèse des acides aminés aromatiques constitue une voie métabolique vitale pour les plantes, les bactéries et les champignons. En plus de la biosynthèse de la tyrosine, de la phénylalanine et du tryptophane, cette voie métabolique joue un rôle essentiel dans la production de nombreux métabolites aromatiques secondaires impliqués dans des processus tels que les interactions plantes-microbes, la biosynthèse de biopolymères structuraux comme la lignine et la subérine, la synthèse hormonale, ou la synthèse des quinones. Parmi l'ensemble des organismes vivants possédant cette voie métabolique, deux voies ont été identifiées pour la transformation du préphénate en tyrosine (Figure 1; Stenmark et al., 1974). Chez la plupart des bactéries chlorophylliennes, quelques microorganismes, et chez la plupart des plantes, la L-tyrosine est synthétisée par la voie de l'arogénate (Abou-Zeid *et al.,* 1995; Byng *et al.,* 1981; Connely and Conn 1986; Frazel and Jensen 1979; Gaines *et al.,* 1982; Hall *et al.,* 1982; Keller *et al.,* 1985; Mayer *et al.,* 1985). Dans cette voie, le préphénate est transaminé en arogénate par une transaminase spécifique, la préphénate aminotransférase (EC 2.6.1.57), puis l'arogénate est transformé en L-tyrosine par une arogénate dehydrogénase (EC 1.3.1.43; ADH sur la Figure 1). D'une manière différente, chez des organismes comme la bactérie *Escherichia coli* ou les levures, le préphénate est dans un premier temps transformé en p-hydroxyphenylpyruvate par une préphénate dehydrogénase (EC 1.3.1.12, EC 1.3.1.13), lequel p-hydroxyphenylpyruvate est transaminé en L-tyrosine (Lingens et al., 1967). De par son rôle dans la voie de biosynthèse de la tyrosine chez les plantes, l'enzyme arogénate dehydrogénase constitue une cible potentielle pour de nouveaux herbicides.

D'autres enzymes impliquées dans cette voie métabolique constituent déjà des cibles d'herbicides majeurs. On peut citer par exemple l'enzyme 5-enolpyruvylshikimate 3-phosphate synthase (EPSPS), intervenant en amont de la synthèse du préphénate, qui est la cible de l'herbicide total glyphosate. On peut également citer l'enzyme p-hydroxyphenylpyruvate dioxygenase (HPPD) impliquée dans la transformation du p-hydroxyphenylpyruvate en homogentisate. L'HPPD est la cible de nouvelles familles d'herbicides dont l'activité conduit au blanchiment des feuilles (Schulz *et al.,* 1993; Secor 1994). Ces herbicides sont notamment les isoxazoles (EP 418 175, EP 470 856, EP 487 352, EP 527 036, EP 560 482, EP 682 659, US 5 424 276) en particulier l'isoxaflutole, herbicide sélectif du maïs, les dicétonitriles (EP 496 630, EP 496 631), en particulier la 2-cyano-3-cyclopropyl-1-(2-SO₂CH₃-4-CF₃ phényl) propane-1,3-dione et la 2-cyano-3-cyclopropyl-1-(2-SO₂CH₃-4-2,3 Cl₂ phényl) propane-1, 3-dione, les tricétones (EP 625 505, EP 625 508, US 5,506,195), en particulier la sulcotrione ou la mésotrione, ou encore les pyrazolinates.

Un des avantages des herbicides ayant pour cible des enzymes impliquées dans les voies métaboliques vitales des plantes est leur large spectre d'activité sur des plantes d'origines phylogénétiques éloignées. Toutefois, de tels herbicides présentent également l'inconvénient majeur, lorsqu'ils sont appliqués sur des cultures pour éliminer les végétaux indésirables ou "mauvaises herbes", d'agir également sur les plantes cultivées. Cet inconvénient peut être pallié par l'utilisation de plantes cultivées tolérantes aux dits herbicides. De telles plantes sont généralement obtenues par génie génétique en introduisant dans leur génome un gène codant une enzyme de résistance audit herbicide de manière à ce qu'elles surexpriment ladite enzyme dans leurs tissus. Jusqu'à présent trois principales stratégies utilisant le génie génétique ont été employée pour rendre des plantes tolérantes aux herbicides. La première consiste à détoxifier l'herbicide par transformation de la plante avec un gène codant une enzyme de détoxification. Cette enzyme transforme l'herbicide, ou son métabolite actif, en produits de dégradation non toxique, comme par exemple les enzymes de tolérance au bromoxynil ou au basta (EP 242 236, EP 337 899). La seconde stratégie consiste à transformer la plante avec un gène codant l'enzyme cible mutée de manière à ce qu'elle soit moins sensible à l'herbicide, ou son métabolite actif, comme par exemple les enzymes de tolérance au glyphosate (EP 293 356, Padgette S. R. & al., J. Biol. Chem., 266, 33, 1991). La troisième stratégie consiste à surexprimer l'enzyme cible sensible, de manière à produire dans la plante des quantités importantes d'enzyme cible, si possible bien supérieures à la quantité d'herbicide pénétrant la plante. Cette stratégie, qui a été employée pour obtenir avec succès des plantes tolérantes aux inhibiteurs d'HPPD (WO 96/38567), permet de maintenir un niveau suffisant d'enzyme fonctionnelle, malgré la présence de son inhibiteur.

Le fait que deux voies de biosynthèse de la L-tyrosine existent dans des groupes taxonomiques différents, et en particulier que la voie transformant directement le préphénate en p-hydroxyphenylpyruvate ne se retrouve pas chez les plantes permet d'envisager une quatrième stratégie pour rendre des plantes tolérantes aux herbicides. En effet, dans le cas de l'utilisation d'un composé herbicide ayant pour cible l'enzyme arogénate déhydrogénase chez les plantes, la transformation des plantes que l'on souhaite rendre tolérantes avec un gène codant une enzyme préphénate déhydrogénase de bactérie ou de levure permettra aux dites plantes de synthétiser de la L-tyrosine, donc de tolérer la présence du composé herbicide, malgré l'inhibition de l'enzyme arogénate déhydrogénase par ledit composé herbicide. Cette nouvelle stratégie consiste donc à créer chez les plantes que l'on souhaite rendre résistantes un contournement de la voie métabolique naturelle de biosynthèse de la tyrosine, laquelle voie utilise l'enzyme arogénate déhydrogénase, par implantation artificielle chez ces plantes d'une nouvelle voie métabolique de biosynthèse de la tyrosine utilisant l'enzyme préphénate déhydrogénase. Un tel contournement permet aux plantes le possédant, de préférence les plantes d'intérêt agronomique, de tolérer la présence du composé herbicide inhibant la voie métabolique naturelle, alors que les plantes ne possédant pas ce contournement, en particulier les végétaux indésirables, seront sensibles audit composé herbicide.

### Description

La présente invention concerne donc de nouveaux polynucléotides isolés codant pour une enzymese possédant une activité arogénate dehydrogénase. Selon la présente invention, on entend par "polynucléotide" une séquence nucléotidique naturelle ou artificielle pouvant être de type ADN ou ARN, de préférence de type ADN, notamment double brin. Par enzymes possédant une activité arogénate dehydrogénase, on entend les enzymes capables de transformer l'arogénate en L-tyrosine. La mesure de l'activité arogenate dehydrogénase est réalisée par toute méthode permettant soit de mesurer la diminution de la quantité du substrat arogénate, soit de mesurer l'accumulation d'un produit issu de la réaction enzymatique, à savoir la L-tyrosine ou le cofacteur NADPH. En particulier, la mesure de l'activité arogenate dehydrogénase peut être réalisée par la méthode décrite à l'exemple 4.

Selon un mode particulier de réalisation de l'invention, les polynucléotides codant pour une enzyme arogénate dehydrogénase comprennent des polynucléotides codant pour la séquence polypeptidique sélectionnée parmi la séquence décrite par l'identificateur de séquence SEQ ID NO:3, SEQ ID NO: 5, SEQ ID NO: 7, SEQ ID NO:9, SEQ ID NO: 11 ou SEQ ID NO:13. Il est bien connu de l'homme du métier que cette définition inclus tous les polynucléotides qui, bien que comprenant des séquences nucléotidiques différentes comme résultat de la dégénérescence du code génétique, codent pour une même séquence d'acides aminés, laquelle est représentée par les identificateurs de séquences SEQ ID NO:3, SEQ ID NO: 5, SEQ ID NO: 7, SEQ ID NO:9, SEQ ID NO: 11 ou SEQ ID NO:13.

La présente invention comprend également des polynucléotides isolés codant pour des enzymes arogénate dehydrogénase et capables de s'hybrider de manière sélective à un des polynucléotides précédemment décrits, ou un fragment de ces polynucléotides constituant une sonde. Par "polynucléotide capable de s'hybrider de manière sélective", on entend selon l'invention les polynucléotides qui, par une des méthodes usuelles de l'état de la technique (Sambrook et al., 1989, Molecular Cloning : A Laboratory Manual, Nolan C. ed., New York: Cold Spring Harbor Laboratory Press), s'hybrident avec les polynucléotides ci-dessus, ou avec les sondes qui en sont dérivées, à un niveau supérieur au bruit de fond de manière significative. Le bruit de fond peut être lié à l'hybridation d'autres polynucléotides présents, par exemple d'autres ADNc présents dans une banque d'ADNc. Le niveau du signal généré par l'interaction entre le polynucléotide capable de s'hybrider de manière sélective et les polynucléotides définis par les séquences SEQ ID NO: ci-dessus selon l'invention, ou les sondes, est généralement 10 fois, de préférence 100 fois plus intense que celui généré par l'interaction avec d'autres séquences d'ADN générant le bruit de fond. Le niveau d'interaction peut être mesuré par exemple, par marquage des polynucléotides décrits ci-dessus ou des sondes avec des éléments radioactifs, comme le ³²P. L'hybridation sélective est généralement obtenue en employant des conditions de milieu *très sévères* (par exemple NaCl 0,03 M et citrate de sodium 0,03 M à environ 50°C-60°C).

L'invention comprend également des polynucléotides isolés codant pour des enzymes arogénate dehydrogénase et homologues des polynucléotides précédemment décrits. Par "homologue", on entend selon l'invention des polynucléotides présentant une ou plusieurs modifications de séquence par rapport aux séquences nucléotidiques décrites ci-dessus et codant pour une enzyme à activité arogénate dehydrogénase fonctionnelle. Ces modifications peuvent être naturelle ou obtenues artificiellement selon les techniques usuelles de mutation conduisant notamment à l'addition, la délétion, ou la substitution d'un ou plusieurs nucléotides par rapport aux séquences de l'invention. Ces modifications déterminent un degré d'homologie vis-à-vis des séquences ci-dessus décrites. De manière avantageuse, le degré d'homologie sera d'au moins 70 % par rapport aux séquences décrites, de préférence d'au moins 80 %, plus préférentiellement d'au moins 90 %. Les méthodes de mesure et d'identification des homologies entre les séquences d'acides nucléiques sont bien connues de l'homme du métier. On peut employer par exemple les programmes PILEUP ou BLAST (Basic Local Alignment Search Tool; Altschul et al., 1993, J. Mol. Evol. 36 :290-300 ; Altschul et al., 1990, J. Mol. Biol. 215 :403-10; voir aussi http://www.ncbi.nlm.nih.gov/BLAST/).

La présente invention concerne également des fragments des polynucléotides décrits ci-dessus. Le terme "fragment" désigne notamment un fragment d'au moins 20 nucléotides, en particulier d'au moins 50 nucléotides, et de préférence d'au moins 100 nucléotides.

Selon un mode particulier de réalisation de l'invention, le polynucléotide selon l'invention est représenté par l'identificateur de séquence SEQ ID NO:1, SEQ ID NO:2, SEQ ID NO:4, SEQ ID NO:6, SEQ ID NO:8, SEQ ID NO:10 ou SEQ ID NO:12.

La présente invention concerne également des polynucléotides comprenant au moins un des polynucléotides tels que décrits précédemment.

Tous les polynucléotides décrits ci-dessus codent des enzymes arogénate dehydrogénase. En conséquence, l'invention s'étend donc à toutes les enzymes arogénate dehydrogénase codées par l'ensemble de ces polynucléotides.

Selon un mode particulier de réalisation de l'invention, l'enzyme arogénate dehydrogénase est une enzyme dont la séquence peptidique est sélectionnée parmi la séquence décrite par l'identificateur de séquence SEQ ID NO:3, SEQ ID NO: 5, SEQ ID NO: 7, SEQ ID NO:9, SEQ ID NO:11 ou SEQ ID NO:13, ou un fragment de ces séquences. Par fragment, on entend essentiellement un fragment biologiquement actif, c'est-à-dire un fragment de la séquence d'une enzyme arogénate dehydrogénase possédant la même activité qu'une enzyme arogénate dehydrogénase complète.

Selon un mode particulier de réalisation de l'invention, les polynucléotides et les enzymes arogénate dehydrogénase décrits ci-dessus proviennent de plantes. Plus particulièrement, ils proviennent de plantes du genre *Arabidopsis,* de préférence du genre *A. thaliana,* ou de plantes du genre *Picea,* de préférence *Picea glauca.*

Selon un autre mode particulier de réalisation de l'invention, les polynucléotides et les enzymes arogénate dehydrogénase décrits ci-dessus proviennent de bactéries. Plus particulièrement, ils proviennent de bactéries du genre *Synechocystis.*

La présente invention concerne également un gène chimère comprenant au moins, liés entre eux de manière opérationnelle, un promoteur fonctionnel dans un organisme hôte, un polynucléotide codant pour une enzyme arogénate dehydrogénase tel que défini dans la présente invention, et un élément terminateur fonctionnel dans ce même organisme hôte. Les différents éléments qu'un gène chimère peut contenir sont, d'une part, des éléments régulateurs de la transcription, de la traduction et de la maturation des protéines, tels qu'un promoteur, une séquence codant pour un peptide signal ou un peptide de transit, ou un élément terminateur constituant un signal de polyadénylation, et d'autre part un polynucléotide codant pour une protéine. L'expression "liés entre eux de manière opérationnelle" signifie que lesdits éléments du gène chimère sont liés entre eux de manière à ce que le fonctionnement d'un de ces éléments est affecté par celui d'un autre. A titre d'exemple, un promoteur est lié de manière opérationnelle à une séquence codante lorsqu'il est capable d'affecter l'expression de ladite séquence codante. La construction du gène chimère selon l'invention et l'assemblage de ses différents éléments est réalisable par l'emploi de techniques bien connues de l'homme du métier, notamment celles décrites dans Sambrook et al. (1989, Molecular Cloning : A Laboratory Manual, Nolan C. ed., New York: Cold Spring Harbor Laboratory Press). Le choix des éléments régulateurs constituant le gène chimère est essentiellement fonction de l'espèce hôte dans laquelle ils doivent fonctionner, et l'homme du métier est capable de sélectionner des éléments régulateurs fonctionnels dans un organisme hôte donné. Par "fonctionnels", on entend capables de fonctionner dans un organisme hôte donné.

Les promoteurs que peut contenir le gène chimère selon l'invention sont soit constitutifs, soit inductibles. Un promoteur constitutif selon la présente invention est un promoteur qui induit l'expression d'une séquence codante dans tous les tissus d'un organisme hôte et en permanence, c'est-à-dire durant toute la durée du cycle vital dudit organisme hôte. Certains de ces promoteurs peuvent être tissu-spécifiques, c'est-à-dire exprimer la séquence codante en permanence, mais uniquement dans un tissu particulier de l'organisme hôte. Des promoteurs constitutifs peuvent provenir de tout type d'organisme. Parmi les promoteurs constitutifs qui peuvent être utilisés dans le gène chimère de la présente invention, nous pouvons citer à titre d'exemple, des promoteurs bactériens, comme celui du gène de l'octopine synthase ou celui du gène de la nopaline synthase, des promoteurs viraux, comme celui du gène contrôlant la transcription des ARN19S ou 35S du virus de la mosaïque du Choux-Fleur (Odell et al., 1985, Nature, 313, 810-812), ou les promoteurs du virus de la mosaïque de la nervure du Manioc (tels que décrits dans la demande de brevet WO 97/48819). Parmi les promoteurs d'origine végétale on citera le promoteur du gène de la petite sous-unité de ribulose-biscarboxylase/oxygénase (RuBisCO), le promoteur d'un gène d'histone tel que décrit dans la demande EP 0 507 698, ou le promoteur d'un gène d'actine de riz (US 5,641,876).

Selon un autre mode particulier de réalisation de l'invention, le gène chimère contient un promoteur inductible. Un promoteur inductible est un promoteur qui ne fonctionne, c'est-à-dire qui n'induit l'expression d'une séquence codante, que lorsqu'il est lui-même induit par un agent inducteur. Cet agent inducteur est en général une substance qui peut être synthétisée dans l'organisme hôte suite à un stimulus externe audit organisme, ce stimulus externe pouvant être par exemple un agent pathogène. L'agent inducteur peut également être une substance externe à cet organisme hôte capable de pénétrer à l'intérieur de celui-ci. De manière avantageuse, le promoteur utilisé dans la présente invention est inductible suite à l'agression de l'organisme hôte par un agent pathogène. De tels promoteurs sont connus, comme par exemple le promoteur du gène d'*O-*méthyltransférase de classe II (COMT II) de plante décrit dans la demande de brevet FR 99 03700, le promoteur PR-1 d'*Arabidopsis* (Lebel et al., 1998, Plant J. 16(2):223-233), le promoteur EAS4 du gène de la sesquiterpène synthase du Tabac (Yin et al., 1997, Plant Physiol. 115(2), 437-451), ou le promoteur du gène codant la 3-hydroxy-3-methylglutaryl coenzyme A reductase (Nelson et al., 1994, Plant Mol. Biol. 25(3):401-412).

Parmi les éléments terminateurs pouvant être utilisés dans le gène chimère de la présente invention, nous pouvons citer à titre d'exemple l'élément terminateur *nos* du gène codant la nopaline synthase *d'Agrobacterium tumefaciens* (Bevan et al., 1983, Nucleic Acids Res. 11(2), 369-385), ou l'élément terminateur d'un gène d'histone tel que décrit dans la demande EP 0 633 317.

Il apparaît également important que le gène chimère comprenne aussi un peptide signal ou un peptide de transit qui permet de contrôler et d'orienter la production de l'enzyme arogénate dehydrogénase de manière spécifique dans une partie de l'organisme hôte, comme par exemple le cytoplasme, un compartiment particulier du cytoplasme, la membrane cellulaire, ou dans le cas des plantes dans un type particulier de compartiments cellulaires, par exemple les chloroplastes, ou dans la matrice extracellulaire.

Les peptides de transit peuvent être soit simples, soit doubles. Les peptides de transit doubles sont éventuellement séparés par une séquence intermédiaire, c'est à dire qu'ils comprennent, dans le sens de la transcription, une séquence codant pour un peptide de transit d'un gène végétal codant pour une enzyme à localisation plastidiale, une partie de séquence de la partie mature N-terminale d'un gène végétal codant pour une enzyme à localisation plastidiale, puis une séquence codant pour un second peptide de transit d'un gène végétal codant pour une enzyme à localisation plastidiale. De tels peptides de transit doubles sont par exemple décrits dans la demande de brevet EP 0 508 909.

Comme peptide signal utile selon l'invention, on peut citer en particulier le peptide signal du gène PR-1α du tabac décrit par Cornelissen et al. (1987, Nucleic Acid Res. 15, 6799-6811) en particulier lorsque le gène chimère selon l'invention est introduit dans des cellules végétales ou des plantes, ou le peptide signal du précurseur du facteur Mat α1 (Brake et al., 1985, In: Gething M.-J. (eds.); Protein transport and secretion, pp.103-108, Cold Spring Harbor Laboratory Press, New York) lorsque le gène chimère selon l'invention est introduit dans des levures.

La présente invention concerne également un vecteur contenant un gène chimère selon l'invention. Le vecteur selon l'invention est utile pour transformer un organisme hôte et exprimer dans celui-ci une enzyme arogénate dehydrogénase. Ce vecteur peut être un plasmide, un cosmide, un bactériophage ou un virus. De manière générale, les principales qualités de ce vecteur doivent être une capacité à se maintenir et à s'autorépliquer dans les cellules de l'organisme hôte, notamment grâce à la présence d'une origine de réplication, et à y exprimer une enzyme arogénate dehydrogénase. Le choix d'un tel vecteur ainsi que les techniques d'insertion dans celui-ci du gène chimère selon l'invention sont largement décrits dans Sambrook et al. (1989, Molecular Cloning : A Laboratory Manual, Nolan C. ed., New York: Cold Spring Harbor Laboratory Press) et font partie des connaissances générales de l'homme du métier. De manière avantageuse, le vecteur utilisé dans la présente invention contient également, en plus du gène chimère de l'invention, un gène codant pour un marqueur de sélection. Ce marqueur de sélection permet de sélectionner les organismes hôtes effectivement transformés, c'est-à-dire ceux ayant incorporé le vecteur. Selon un mode particulier de réalisation de l'invention, l'organisme hôte à transformer est un microorganisme, en particulier une levure, une bactérie, un champignon, ou un virus. Selon un autre mode de réalisation, l'organisme hôte est une plante ou une cellule végétale. Parmi les gènes codant pour des marqueurs de sélection utilisables, on peut citer les gènes de résistance aux antibiotiques tel que, par exemple, le gène de l'hygromycine phosphotransférase (Gritz et al., 1983, Gene 25:179-188), mais également les gènes de tolérance aux herbicides tel que le gène *bar* (White et al., NAR 18:1062, 1990) pour la tolérance au bialaphos, le gène EPSPS (US 5,188,642) pour la tolérance au glyphosate ou encore le gène HPPD (WO 96/38567) pour la tolérance aux isoxazoles. On peut également citer des gènes codant pour des enzymes facilement identifiables comme l'enzyme GUS, des gènes codant pour des pigments ou des enzymes régulant la production de pigments dans les cellules transformées. De tels gènes marqueurs de sélection sont notamment décrits dans les demandes de brevet WO 91/02071, WO 95/06128, WO 96/38567, et WO 97/04103.

La présente invention concerne également des organismes hôte transformés, contenant un vecteur tel que décrit ci-dessus. Par organisme hôte, on entend tout organisme mono ou pluricellulaire, inférieur ou supérieur, dans lequel le gène chimère selon l'invention peut être introduit, pour la production d'enzyme arogénate dehydrogénase. Il s'agit en particulier de bactéries, par exemple *Escherichia coli,* de levures, en particulier des genres *Saccharomyces, Kluyveromyces,* ou *Pichia,* de champignons, en particulier *Aspergillus,* d'un baculovirus, ou de préférence de cellules végétales et de plantes.

Par "cellule végétale", on entend selon l'invention toute cellule issue d'une plante et pouvant constituer des tissus indifférenciés tels que des cals, des tissus différenciés tels que des embryons, des parties de plantes, des plantes ou des semences.

On entend par "plante" selon l'invention, tout organisme multicellulaire différencié capable de photosynthèse, en particulier monocotylédones ou dicotylédones.

On entend par "organisme hôte transformé", un organisme hôte qui a incorporé dans son génome le gène chimère de l'invention, et produit en conséquence une enzyme arogénate dehydrogénase dans ses tissus, ou dans un milieu de culture. Pour obtenir les organismes hôtes selon l'invention, l'homme du métier peut utiliser une des nombreuses méthodes de transformation connues.

Une de ces méthodes consiste à mettre les cellules à transformer en présence de polyéthylène glycol (PEG) et des vecteurs de l'invention (Chang and Cohen, 1979, Mol. Gen. Genet. 168(1), 111-115; Mercenier and Chassy, 1988, Biochimie 70(4), 503-517). L'électroporation est une autre méthode qui consiste à soumettre les cellules ou tissus à transformer et les vecteurs de l'invention à un champ électrique (Andreason and Evans, 1988, Biotechniques 6(7), 650-660; Shigekawa and Dower, 1989, Aust. J. Biotechnol. 3(1),56-62). Une autre méthode consiste à directement injecter les vecteurs dans les cellules ou les tissus hôtes par micro-injection (Gordon and Ruddle, 1985, Gene 33(2), 121-136). De manière avantageuse, la méthode dite de "biolistique" pourra être utilisée. Elle consiste à bombarder des cellules ou des tissus avec des particules sur lesquelles sont adsorbés les vecteurs de l'invention (Bruce et al., 1989, Proc. Natl. Acad. Sci. USA 86(24), 9692-9696; Klein et al., 1992, Biotechnology 10(3), 286-291; US Patent No. 4,945,050). De manière préférentielle, la transformation de plantes se fera à l'aide de bactéries du genre *Agrobacterium,* de préférence par infection des cellules ou tissus desdites plantes par *A. tumefaciens* (Knopf, 1979, Subcell. Biochem. 6, 143-173; Shaw et al., 1983, Gene 23(3):315-330) *ou A. rhizogenes* (Bevan et Chilton, 1982, Annu. Rev. Genet. 16:357-384; Tepfer and Casse-Delbart, 1987, Microbiol. Sci. 4(1), 24-28). De manière préférentielle, la transformation de cellules végétales par *Agrobacterium tumefaciens* est réalisée selon le protocole décrit par Ishida et al. (1996, Nat. Biotechnol. 14(6), 745-750).

L'homme du métier fera le choix de la méthode appropriée en fonction de la nature de l'organisme hôte à transformer.

La présente invention concerne donc également un procédé de préparation de l'enzyme arogénate dehydrogénase, comprenant les étapes de culture d'un organisme hôte transformé comprenant un gène codant pour une enzyme arogénate dehydrogénase tel que défini ci-dessus dans un milieu de culture approprié, de récupération de l'enzyme arogenate dehydrogénase produite dans le milieu de culture par centrifugation ou par filtration, puis de purification de l'enzyme récupérée par un passage au travers d'au moins une colonne de chromatographie. Ces étapes conduisent à l'extraction et à la purification totale ou partielle de l'enzyme arogénate dehydrogénase obtenue. De manière préférentielle, l'organisme transformé est un microorganisme, en particulier une bactérie, une levure, un champignon, ou un virus.

La présente invention comprend également un procédé d'identification d'un composé herbicide ayant pour cible une enzyme arogenate dehydrogénase, caractérisé en ce que:
(a) on prépare au moins deux échantillons contenant chacun une quantité équivalente d'enzymes arogenate dehydrogénase en solution
(b) on traite un des échantillons avec un composé
(c) on mesure l'activité arogenate dehydrogénase dans chacun desdits échantillons
(d) on identifie le composé utilisé à l'étape (b) comme étant un composé herbicide lorsque l'activité mesurée à l'étape (c) est significativement inférieure dans l'échantillon traité par rapport à l'échantillon non traité
(e) on valide l'activité herbicide du composé identifié à l'étape (d) en traitant des plantes avec ledit composé

Selon le présent procédé, la mesure de l'activité arogenate dehydrogénase est réalisée par toute méthode permettant soit de mesurer la diminution de la quantité du substrat arogénate, soit de mesurer l'accumulation d'un produit issu de la réaction enzymatique, à savoir la L-tyrosine ou le cofacteur NADPH. En particulier, la mesure de l'activité arogenate dehydrogénase peut être réalisée par la méthode décrite à l'exemple 4. De plus, l'activité herbicide validée à l'étape (e) du présent procédé peut être une activité mortelle se traduisant par la mort de la plante traitée, ou une activité de ralentissement significatif de la croissance de la plante traitée.

Par composé, on entend selon l'invention tout composé chimique ou mélange de composés chimiques, y compris les peptides et les protéines. Par mélange de composés on comprend selon l'invention au moins deux composés différents, comme par exemple les (dia)stéréoisomères d'une molécule, des mélanges d'origine naturelle issus de l'extraction de matériel biologique (plantes, tissus végétaux, culture bactériennes, cultures de levures ou de champignons, insectes, tissus animaux, etc.) ou des mélanges réactionnels non purifiés ou purifiés totalement ou en partie, ou encore des mélanges de produits issus de techniques de chimie combinatoire.

Selon un mode particulier de réalisation du procédé selon l'invention, les enzymes arogénate dehydrogénase employées proviennent de plantes, de préférence *d'Arabidopsis thaliana.*

Selon un autre mode de réalisation du procédé selon l'invention, les enzymes arogénate dehydrogénase employées proviennent de bactéries, de préférence de bactéries du genre *Synechocystis.*

De préférence, les enzymes arogénate dehydrogénase employées dans le procédé selon l'invention sont les enzymes selon la présente invention, en particulier celles représentées par les SEQ ID NO:3, SEQ ID NO: 5, SEQ ID NO: 7, SEQ ID NO:9, SEQ ID NO:11 ou SEQ ID NO:13.

L'invention s'étend également aux composés herbicides identifiés par le procédé ci-dessus mentionné, en particulier les composés herbicides ayant pour cible une enzyme arogénate dehydrogénase, c'est-à-dire ceux qui inhibent l'activité de cette enzyme. De manière préférentielle, les composés herbicides ne sont pas des inhibiteurs généraux d'enzymes. De manière également préférentielle les composés herbicides selon l'invention ne sont pas des composés déjà connus pour avoir une activité herbicide.

La présente invention concerne également des compositions agrochimiques herbicides comprenant comme matière active au moins une quantité efficace d'un composé herbicide selon l'invention.

Par composition agrochimique herbicide, on entend selon l'invention une composition applicable de manière préventive ou curative sur les surfaces sur lesquelles des végétaux cultivés sont ou doivent être cultivés, afin d'empêcher le développement de végétaux indésirables ou "mauvaises herbes" sur les surfaces sur lesquelles sont mis en culture lesdits végétaux cultivés, quel que soit leur stade de développement. Une quantité efficace de composé herbicide selon l'invention correspond à une quantité de composé permettant de détruire ou d'inhiber la croissance des végétaux indésirables.

Les compositions agrochimiques herbicides selon l'invention comprennent un composé herbicide selon l'invention ou un de ses sels acceptables en agriculture ou un complexe métallique ou métalloïdique de ce composé, en association avec un support solide ou liquide, acceptable en agriculture et/ou un agent tensioactif également acceptable en agriculture. En particulier sont utilisables les supports inertes usuels et les agents tensioactifs usuels. Ces compositions recouvrent non seulement les compositions prêtes à être appliquées sur une plante ou une semence à traiter au moyen d'un dispositif adapté, tel qu'un dispositif de pulvérisation ou de poudrage, mais également les compositions concentrées commerciales qui doivent être diluées avant application sur la culture.

Les compositions herbicides selon l'invention peuvent contenir aussi de nombreux autres ingrédients tels que, par exemple, des colloïdes protecteurs, des adhésifs, des épaississants, des agents thixotropes, des agents de pénétration, des stabilisants, des séquestrants. Plus généralement, les matières actives peuvent être combinées à tous les additifs solides ou liquides correspondant aux techniques habituelles de la mise en formulation.

Par le terme "support", on désigne selon la présente invention une matière organique ou minérale, naturelle ou synthétique, avec laquelle la matière active est combinée pour faciliter son application sur les parties de la plante. Ce support est donc généralement inerte et il doit être acceptable en agriculture. Le support peut être solide (par exemple des argiles, silicates naturels ou synthétiques, silice, résines, cires, engrais solides) ou liquide (par exemple de l'eau, des alcools, notamment le butanol).

L'agent tensioactif peut être un agent émulsionnant, dispersant ou mouillant de type ionique ou non ionique ou un mélange de tels agents tensioactifs. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés, des esters d'acides gras et de polyols, les dérivés à fonction sulfates, sulfonates et phosphates des composés précédents. La présence d'au moins un agent tensioactif est généralement indispensable lorsque la matière active et/ou le support inerte ne sont pas solubles dans l'eau et que l'agent vecteur de l'application est l'eau.

La présente invention concerne également des plantes transgéniques tolérantes à un composé herbicide ayant pour cible une enzyme impliquée dans l'une des étapes métaboliques de transformation du préphénate en L-tyrosine, caractérisées en ce qu'elles contiennent un gène codant une enzyme préphénate dehydrogénase et expriment ladite enzyme dans leurs tissus. Une enzyme préphénate dehydrogénase est une enzyme catalysant la réaction de transformation du préphénate en p-hydroxyphénylpyruvate. L'identification d'une enzyme à activité préphénate dehydrogénase peut être réalisée par toute méthode permettant soit de mesurer la diminution de la quantité du substrat préphénate, soit de mesurer l'accumulation d'un produit issu de la réaction enzymatique, à savoir le p-hydroxyphénylpyruvate ou un des cofacteurs NADH ou NADPH. En particulier, la mesure de l'activité préphénate dehydrogénase peut être réalisée par la méthode décrite à l'exemple 4.

Selon un mode particulier de réalisation de l'invention, les plantes transgéniques selon l'invention sont tolérantes vis-à-vis d'un composé herbicide ayant pour cible une enzyme arogénate déhydrogénase, de préférence une enzyme arogénate déhydrogénase telle que décrite dans la présente invention.

Selon un autre mode particulier de réalisation de l'invention, les plantes transgéniques selon l'invention sont tolérantes vis-à-vis d'un composé herbicide ayant pour cible une enzyme préphénate aminotransférase.

Selon un mode particulier de réalisation de l'invention, le gène codant l'enzyme préphénate dehydrogénase exprimée dans les plantes tolérantes selon l'invention est un gène de levure. De préférence, il s'agit du gène codant l'enzyme préphénate dehydrogénase de *Saccharomyces cerevisiae* (No. d'accession NC001134) tel que décrit dans Mannhaupt et al. (1989, Gene 85, 303-311) et représenté par l'identificateur de séquence SEQ ID NO: 14.

Selon un autre mode particulier de réalisation de l'invention, le gène codant l'enzyme préphénate dehydrogénase exprimée dans les plantes tolérantes selon l'invention est un gène de bactérie. De préférence, il s'agit d'un gène de bactérie du genre *Bacillus,* en particulier de l'espèce *B. subtilis* (No. d'accession M80245) tel que représenté par l'identificateur de séquence SEQ ID NO:16. De préférence, il s'agit d'un gène de bactérie du genre *Escherichia,* en particulier de l'espèce *E. coli* (No. d'accession M10431) telle que décrit dans Hudson et al. (1984, J. Mol. Biol. 180(4), 1023-1051) et représenté par l'identificateur de séquence SEQ ID NO: 18. De préférence, il s'agit d'un gène de bactérie du genre *Erwinia,* en particulier de l'espèce *d'E. herbicola* (No. d'accession 43343) tel que représenté par l'identificateur de séquence SEQ ID NO: 20.

Selon un mode particulier de réalisation de l'invention, le gène codant l'enzyme préphénate dehydrogénase exprimée dans les plantes tolérantes selon l'invention est un gène de champignon.

Les plantes transgéniques selon l'invention sont obtenues par transformation génétique avec un gène codant une enzyme préphénate dehydrogénase. De préférence, ce gène est un gène chimère comprenant au moins, liés entre eux de manière opérationnelle, un promoteur fonctionnel dans un organisme hôte, un polynucléotide codant pour une enzyme préphénate dehydrogénase, et un élément terminateur fonctionnel dans ce même organisme hôte. Ce gène est généralement introduit dans un vecteur, lequel est utilisé pour introduire ledit gène dans lesdites plantes par une des méthodes de transformation décrites ci-dessus.

La présente invention concerne également un procédé de production de plantes tolérantes vis-à-vis de composés herbicides ayant pour cible une enzyme impliquée dans l'une des étapes métaboliques de transformation du préphénate en L-tyrosine, caractérisé en ce que l'on transforme lesdites plantes avec un gène codant une enzyme préphénate dehydrogénase de manière à ce qu'elles l'expriment dans leurs tissus.

Selon un mode particulier de réalisation de l'invention, le présent procédé s'applique à la production de plantes tolérantes vis-à-vis d'un composé herbicide ayant pour cible une enzyme arogénate déhydrogénase telle que décrite dans la présente invention.

Selon un autre mode particulier de réalisation de l'invention, le présent procédé s'applique à la production de plantes tolérantes vis-à-vis d'un composé herbicide ayant pour cible une enzyme préphénate aminotransférase.

Le présent procédé comprend donc également un procédé de production de plantes tolérantes vis-à-vis d'un composé herbicide ayant pour cible une enzyme arogénate déhydrogénase, caractérisé en ce que l'on transforme lesdites plantes avec un gène codant une enzyme préphénate dehydrogénase de manière à ce qu'elles l'expriment dans leurs tissus.

Les plantes transgéniques selon l'invention peuvent également contenir, en plus d'un gène codant une enzyme préphénate dehydrogénase, au moins un autre gène contenant un polynucléotide codant pour une protéine d'intérêt. Parmi les polynucléotides codant pour une protéine d'intérêt, on peut citer des polynucléotides codant une enzyme de résistance à un herbicide, par exemple le polynucléotide codant pour l'enzyme *bar* (White et al., NAR 18:1062, 1990) pour la tolérance au bialaphos, le polynucléotide codant pour l'enzyme EPSPS (US 5,188,642; WO 97/04103) pour la tolérance au glyphosate ou encore le polynucléotide codant pour l'enzyme HPPD (WO 96/38567) pour la tolérance aux isoxazoles. On peut également citer un polynucléotide codant pour une toxine insecticide, par exemple un polynucléotide codant pour une toxine de la bactérie *Bacillus thuringiensis* (pour exemple, voir International Patent Application WO 98/40490). Peuvent également être contenus dans ces plantes d'autres polynucléotides de résistance aux maladies, par exemple un polynucléotide codant pour l'enzyme oxalate oxydase tel que décrit dans la demande de brevet EP 0 531 498 ou le brevet US 5,866,778, ou un polynucléotide codant pour un autre peptide antibactérien et/ou antifongique tels que ceux décrits dans les demandes de brevets WO 97/30082, WO 99/24594, WO 99/02717, WO 99/53053, et WO99/91089. On peut également citer des polynucléotides codant pour des caractères agronomiques de la plante, en particulier un polynucléotide codant pour une enzyme delta-6 désaturase tel que décrit dans les brevets US 5,552,306, US 5,614,313, et demandes de brevets WO 98/46763 et WO 98/46764, ou un polynucléotide codant pour une enzyme sérine acétyltransférase (SAT) tel que décrit dans les demandes de brevets WO 00/01833 et PCT/FR 99/03179.

Les exemples ci-après permettent d'illustrer la présente invention, sans toutefois en limiter la portée.

### Exemple 1: Identification du gène codant l'enzyme arogénate déhydrogénase d'Arabidpsis thaliana

Une comparaison des séquences de toutes les enzymes préphénate déhydrogénase et arogénate déhydrogénase actuellement disponibles dans les bases de données publiques (http://www.ncbi.nlm.nih.gov) a révélé quatre courtes portions de séquences homologues. Les enzymes comparées sont la préphénate déhydrogénase de levure (numéro d'accession: Z36065), la préphénate déhydrogénase de *Bacillus subtilis* (numéro d'accession: M80245), et la préphénate déhydrogénase de *Synechocystis* (numéro d'accession: D90910). Ces portions d'homologie ont permis d'identifier un gène d*'A. thaliana* (numéro d'accession: AF096371) initialement annoté comme codant une enzyme "similaire à la D-isomer spécifique 2-hydroxyacid dehydrogénase". Ce gène est constitué de deux exons séparés par un intron de 94 bp. Le premier exon comporte un cadre ouvert de lecture de 1.08 kb contenant une séquence putative de peptide de transit chloroplastique située en aval du premier codon ATG. Le second exon code potentiellement pour un cadre ouvert de lecture de 892 bp. Une très forte homologie d'environ 60 % existe entre les séquences protéiques déduites des deux exons. Cette homologie s'étend à 70% si la séquence putative de peptide de transit chloroplastique située dans le premier exon n'est pas prise en compte. De plus, chacune des deux séquences protéiques prédites a la taille et possède les quatre portions homologues caractéristiques des enzymes préphénate/arogénate déhydrogénases. Ce gène a été appelé *TyrA* (SEQ ID NO:1).

### Exemple 2: Caractérisation transcriptionnelle de TyrA

La taille du transcrit du gène TyrA a été déterminée par les techniques de Northern Blot et PCR. Des ARNm purifiés extraits de jeunes feuilles *dA. thaliana* ont été hybridées avec des sondes radiomarquées au ³²P correspondant à des fragments d'ADN des deux exons de *TyrA.* Cette analyse a permis d'identifier un transcrit de 1.8-1.9 kb très proche de la taille présumée d'un ARNm contenant les deux exons. De plus, bien que l'ADNc complet n'aie pas pu être amplifié par PCR, un fragment PCR de 1.5 kb a été obtenu. Ce fragment comprend l'oligonucléotide 5' (P8 = 5'-GCTAAAACTCTTCTCCTTCAATACTTACCTG-3') commençant en position 513 bp, et l'oligonucléotide 3' (P7 = 5'-CAGTATAATTAGTAGTCAAGGATCCTGACTGAGAG-3') complémentaire du 3'UTR et commençant en position 2053 bp. Ce fragment contient une partie de la première séquence codante (*TyrA-AT1*) et la séquence complète de la seconde séquence codante (*TyrA-AT2*). L'analyse de la séquence de cet ADNc a confirmé l'épissage de l'intron. Les résultats des analyses par Northern Blot et PCR suggèrent fortement l'existence d'un transcrit ARNm contenant les deux régions codantes *TyrA-AT1* (SEQ ID NO:4) et *TyrA-AT2* (SEQ ID NO:6).

### Exemple 3: Préparation de construits contenant les différentes séquences codantes de l'arogénate déhydrogénase d'A. thaliana

Le premier exon *TyrA-AT1* a été obtenu par amplification PCR de l'ADN génomique d'*A. thaliana* avec l'oligonucléotide P1 (5'-TCTCCATATGATCTTTCAATCTCATTCTCATC-3') qui introduit un site de restriction *Nde* I (souligné) au niveau du premier codon ATG, et l'oligonucléotide P2 (5'-CTAACTAACTAACTACATACCTCATCATATCC-3') qui est complémentaire de l'extrémité 3' du premier exon et de l'extrémité 5' de l'intron et introduit un codon stop (souligné). Trois construits exempts de la séquence codant le peptide de transit ont également été réalisés avec l'oligonucléotide P3 (5'-CCTCTCTTTCCATATGCTCCCTTCTC-3') qui introduit un site de restriction *Nde* I (souligné) au niveau du second codon ATG (M43) en position 127, l'oligonucléotide P4 (5'-CCGCCAGCCACCTCCATATGACCGACACCATCC- 3') qui introduit un codon initiateur ATG et un site de restriction *Nde* I (souligné) en position 174 depuis le premier codon ATG (V58M), et l'oligonucléotide P5 (5'-CGCCACCCCTCATATGCGTATCGCC-3') qui introduit un codon initiateur ATG et un site de restriction *Nde* I (souligné) en position 222 depuis le premier codon ATG (L75M). Tous les fragments PCR correspondant au premier exon, codant un peptide de transit ou non, ont été clonés dans le plasmide pPCR-Script (Stratagène). Des fragments d'ADN Nde I-BamH I contenant les séquences codantes, avec ou sans la séquence de peptide de transit, ont ensuite été clonés dans le plasmide pET21 a(+) (Novagen), conduisant à l'élaboration des plasmides pET21*-TyrA-AT1,* avec et sans séquence de peptide de transit (pET21*-TyrA-AT1-*M1, pET21-*TyrA*-A*T1-*M43, pET21*-TyrA-AT1-*M58, et pET21-*TyrA-AT1-*M75)*.*
Deux autres oligonucléotides ont été utilisés pour amplifier la deuxième séquence codante *(TyrA-AT2).* L'oligonucléotide P6 (5'-GATGCATCTTTGCATATGATGAGGTCAGAAGATG-3') introduit un site de restriction *Nde* I (souligné) au niveau du codon ATG du second cadre ouvert de lecture (en position 1081 depuis le premier codon ATG), et l'oligonucléotide P7 (5'-CAGTATAATTAGTAGTCAAGGATCCTGACTGAGAG-3') complémentaire du début du 3'-UTR qui introduit un site de restriction *BamH* I (souligné). Le fragment PCR correspondant à la deuxième séquence codante a été digéré par *Nde* I*-BamH* I*,* puis cloné dans le plasmide pET21 a(+), donnant la plasmide pET21*-TyrA-AT2.*
La séquence codante complète a été reconstituée par assemblage de l'extrémité 5' manquante du premier exon avec un ADNc de *TyrA-AT* partiel (1.5 kb), obtenu par amplification PCR de l'ADNc d'*Arabidopsis* avec l'oligonucléotide P8 (5'-GCTAAAACTCTTCTCCTTCAATACTTACCTG-3') commençant en position 513 bp depuis le premier codon ATG et l'oligonucléotide 3' P7. Un site de restriction *Eco*RV situé en position 812 bp depuis le premier codon ATG et présent dans l'extrémité 5' de l'ADNc partiel de *TyrA-AT* a été utilisé pour la reconstitution. L'ADNc partiel de *TyrA -AT* a été cloné dans le plasmide pPCR-Script. Un fragment *Eco*RV-*Eco*RV a été obtenu du plasmide pPCR-Script-*TyrA-AT* puis cloné dans le plasmide pPCR-Script-*TyrA-AT1* préalablement digéré par *Eco*RV. Cette manipulation a conduit à l'obtention du plasmide pPCR-Script-*TyrA*-*ATc*. Un fragment Nde1-BamH1 contenant la séquence codante complète a été excisé du plasmide pPCR-Script-*TyrA*-*ATc*, puis cloné dans un plasmide pET21a(+) (Novagen), préalablement digéré par Nde1 et BamH1, conduisant au plasmide pET21a(+)*-TyrA-ATc.* Puis, de la même manière que pour le premier exon, quatre plasmides pET21a(+)-*TyrA-AT*c ont été obtenus. Un plasmide contenant la séquence codante complète avec la séquence codant le peptide de transit putatif, et trois plasmides dénués de cette séquence de peptide de transit qui a été clivée à trois sites différents (M43, V58, et L75, voir ci-dessus).
Pour tous les construits décrits ci-dessus, les inserts d'ADNc ont été séquençés afin de s'assurer qu'aucune mutation indésirable n'a été introduite qu cours des amplifications PCR.

### Exemple 4: Mesure des activités enzymatiques

L'activité arogénate deshydrogénase est mesurée à 25°C par suivi spectrophotométrique à 340 nm de la formation de NADH ou NADPH dans une solution contenant 50 mM de Tris-HCl, pH 8.6, 300 µM d'arogénate, et 1 mM de NAD ou NADP dans un volume total de 200 µl.

L'activité préphénate deshydrogénase est mesurée à 25°C par suivi spectrophotométrique à 340 nm de la formation de NADH ou NADPH dans une solution contenant 50 mM de Tris-HCl, pH 8.6, 300 µM de préphénate, et 1 mM de NAD ou NADP dans un volume total de 200 µl.

### Exemple 5: Production d'arogénate déhydrogénase recombinante

Des cellules *d'Escherichia coli* AT2471 ont été transformées avec chacun des plasmides *pET21-TyrA-AT* obtenus à l'exemple 3, puis cultivée à 37°C dans 2 litres de milieu Luria-Bertani supplémenté avec 100 µg/ml de carbeniciline. Lorsque la culture a atteint l'équivalent d'une absorbance à 600nm (A600) de 0,6, 1 mM d'isopropyl-β-D-thiogalactoside a été ajouté au milieu de culture afin d'induire le synthèse des protéines recombinantes. Les cellules ont ensuite été cultivées pendant 16h à 28°C, récoltées, puis centrifugées 20 min à 40 000 g. Le culot a ensuite été resuspendu dans un tampon Tris-HCl 50 mM, pH 7.5, 1 mM EDTA, 1 mM dithiothreitol, 1 mM benzamidine HCl, 5 mM amino caproic acid, puis soniqué (100 impulsions toutes les 3 secondes à la puissance 5) avec un disrupteur Vibra-Cell (Sonics and Materials, Danbury, CT, USA). Les extraits bruts ainsi obtenus sont ensuite centrifugés 20 min à 40 000 g, et les surnageant sont utilisés directement pour les tests enzymatiques.
Les analyses par SDS-PAGE d'extraits protéiques totaux de la souche *E. coli* AT 2471 contenant les différents construits p*E*T21*-TyrA-Atc,* pET21*-TyrA-AT1,* et p*E*T21*-TyrA-AT2,* ont révélé la présence de trois protéines recombinantes possédant des masses moléculaires de 66-68 kDa, 35 kDa, et 33-34 kDa respectivement. Ces masses moléculaires correspondent bien avec les masses déduites de leurs séquences codantes respectives (68786 Da pour TyrA-ATc, 34966 Da pour Tyr-A-AT1, et 34069 Da pour Tyr-A-AT2). Pour les transformants contenant la séquence codante complète (*TyrA-Atc*) et la première séquence codante (*TyrA-AT1*), des protéines recombinantes n'ont été observées qu'avec les construits codant les protéines M58-TyrA-Atc et M58-TyrA-AT1. Les trois protéines recombinantes ont principalement été retrouvées dans les corps protéiques. Toutefois, la présence de faibles quantités de protéines recombinantes dans les extraits protéiques solubles d'*E*. *coli* ont permis une caractérisation de leurs propriétés biochimiques.

### Exemple 6: Identification et caractérisation biochimique des enzymes arogénate dehydrogénase d'Arabidopsis thaliana

La caractérisation biochimique des enzymes arogénate déhydrogénase recombinantes a été mise en oeuvre à partir des extraits protéiques solubles des souches d'*E*.*coli* transformées. L'activité arogénate déhydrogénase a été mesurée selon la méthode décrite à l'exemple 4. Une activité arogénate déhydrogénase strictement NADP-dépendante a été mise en évidence pour chacune des trois enzymes recombinantes. Aucune activité arogénate déhydrogénase n'a été détectée en présence de NAD, et aucune activité préphénate déhydrogénase n'a été détectée quel que soit le cofacteur utilisé (NADP ou NAD) et quelle que soit la protéine testée (TyrA-ATc, TyrA-AT1, ou TyrA-AT2). De plus, le préphénate à une concentration de 1 mM n'inhibe pas l'activité arogénate déhydrogénase des trois enzymes recombinantes. Chacune de ces enzymes a un comportement de type Michaelis-Menten, et leur valeur de Km pour l'arogénate et le NADP est peu différent (Figures 2 et 3). Les constantes de Michaelis pour le NADP sont respectivement de 40 µM pour TyrA-Atc, 60 µM pour TyrA-AT1, et 20 µM pour TyrA-AT2. Les constantes de Michaelis pour l'arogénate sont respectivement de 70 µM pour TyrA-Atc, 45 µM pour TyrA-AT1, et 45 µM pour TyrA-AT2. De plus, comme les autres arogénates déhydrogénases de plantes (Byng et al., 1981, Phytochemistry 6, 1289-1292; Connelly and Conn, 1986, Z. Naturforsch 41c, 69-78; Gaines et al., 1982Planta 156, 233-240), les arogénates déhydrogénases *d'Arabidopsis* sont toutes très sensibles à la tyrosine, le produit de la réaction enzymatique, et insensibles à 1 mM de phenylalanine et 1 mM de p-hydroxyphenylpyruvate. L'inhibition par la tyrosine est compétitive vis-à-vis de l'arogénate (Ki de 14 µM pour TyrA-Atc, 8 µM pour TyrA-AT1, et 12 µM pour TyrA-AT2), et non-compétitive vis-à-vis du NADP.

### Exemple 7: Identification et caractérisation biochimique de l'enzyme arogénate dehydrogénase de Synechocystis

La séquence du gène codant l'arogénate déhydrogénase *d'A. thaliana* identifiée à l'exemple 1 (*TyrA*) a permis d'identifier un gène d'arogénate déhydrogénase chez la bactérie *Synechocystis* (numéro d'accession: 1652956). Ce gène était originellement décrit comme codant une enzyme "préphénate déhydrogénase". Il a été isolé à partir de d'une banque génomique de *Synechocystis,* et l'enzyme a été produite de la même manière que l'enzyme d'*A*. *thaliana* selon le protocole décrit à l'exemple 5. Une caractérisation biochimique de l'enzyme produite a permis de démontrer qu'il s'agit d'une enzyme arogénate déhydrogénase et non pas d'une enzyme préphénate déhydrogénase. Cette caractérisation biochimique de l'enzyme arogénate déhydrogénase de *Synechocystis* a été mise en oeuvre à partir des extraits protéiques solubles purifiés des souches d*'E.coli* transformées. L'activité arogénate déhydrogénase a été mesurée selon la méthode décrite à l'exemple 4. Une activité arogénate déhydrogénase strictement NADP-dépendante a été mise en évidence pour cette enzyme. Aucune activité arogénate déhydrogénase n'a été détectée en présence de NAD, et aucune activité préphénate déhydrogénase n'a été détectée quel que soit le cofacteur utilisé (NADP ou NAD). De plus, le préphénate à une concentration de 1 mM n'inhibe pas l'activité arogénate déhydrogénase de cette enzyme. L'arogénate déhydrogénase de *Synechocystis* a un comportement de type Michaelis-Menten (Figure 4). La constante de Michaelis est de 6 µM pour le NADP, et de 107 µM pour l'arogénate.

### Exemple 8: Identification d'autres enzymes arogénate déhydrogénase de plantes

La séquence du gène codant l'arogénate déhydrogénase *d'A. thaliana* identifiée à l'exemple 1 (*TyrA*) a permis d'identifier un autre gène d'arogénate déhydrogénase chez *A. thaliana.* Ce nouveau gène (Numéro d'accession: AC034256; SEQ ID NO:8) était initialement annoté comme "contenant une similarité avec la protéine d'abondance de l'embryon (EMB20) de *Picea glauca".* Il possède également une séquence de peptide de transit chloroplastique putative, mais pas de région répétée.

La séquence du gène *TyrA* a également permis d'identifier deux autres ADNc codant pour des enzymes arogénate déhydrogénase dans les bases de données d'EST (Expressed Sequence Tags) publiques. Une de ces ADNc, qui n'est pas complet, correspond à un ADNc de Tomate (TC41067; SEQ ID NO:22). Le caractère incomplet de cet ADNc ne permet pas de déterminer s'il est dupliqué comme TyrA car son extrémité 3' s'arrête juste après le codon correspondant au D356 de Tyr-AT1. Le second ADNc correspond à un ADNc complet de *Picea glauca* (Numéro d'accession: L47749; SEQ ID NO:10), et ne possède pas de région répétée. Cet ADNc de *Picea glauca* était annoté comme étant une protéine d'abondance de l'embryon".

## Revendications

1. Plantes tolérantes vis-à-vis d'un composé herbicide ayant pour cible une enzyme impliquée dans l'une des étapes métaboliques de transformation du préphénate en L-tyrosine, **caractérisé en ce qu'**elles contiennent un gène codant une enzyme préphénate dehydrogénase et expriment ladite enzyme dans leurs tissus

2. Plantes selon la revendication 1, **caractérisées en ce qu'**elles sont tolérantes vis-à-vis d'un composé herbicide ayant pour cible une enzyme arogénate déhydrogénase

3. Plantes selon la revendication 1, **caractérisées en ce qu'**elles sont tolérantes vis-à-vis d'un composé herbicide ayant pour cible une enzyme préphénate aminotransférase

4. Plantes tolérantes selon l'une revendications 1 à 3, **caractérisées en ce que** le gène codant une enzyme préphénate dehydrogénase est un gène de levure

5. Plantes tolérantes selon la revendication 4, **caractérisées en ce que** le gène codant une enzyme préphénate dehydrogénase provient d'une levure du genre *Saccharomyces*

6. Plantes tolérantes selon la revendication 5, **caractérisées en ce que** le gène codant une enzyme préphénate dehydrogénase est représenté par l'identificateur de séquence SEQ ID NO:14

7. Plantes tolérantes l'une revendications 1 à 3, **caractérisées en ce que** le gène codant une enzyme préphénate dehydrogénase provient d'une bactérie

8. Plantes tolérantes selon la revendication 7, **caractérisées en ce que** le gène codant une enzyme préphénate dehydrogénase provient d'une bactérie du genre *Bacillus*

9. Plantes tolérantes selon la revendication 8, **caractérisées en ce que** le gène codant une enzyme préphénate dehydrogénase est représenté par l'identificateur de séquence SEQ ID NO:16

10. Plantes tolérantes selon la revendication 7, **caractérisées en ce que** le gène codant une enzyme préphénate dehydrogénase provient d'une bactérie du genre *Escherichia*

11. Plantes tolérantes selon la revendication 10, **caractérisées en ce que** le gène codant une enzyme préphénate dehydrogénase est représenté par l'identificateur de séquence SEQ ID NO:18

12. Plantes tolérantes selon la revendication 7, **caractérisées en ce que** le gène codant une enzyme préphénate dehydrogénase provient d'une bactérie du genre *Erwinia*

13. Plantes tolérantes selon la revendication 12, **caractérisées en ce que** le gène codant une enzyme préphénate dehydrogénase est représenté par l'identificateur de séquence SEQ ID NO:20

14. Procédé de production de plantes tolérantes vis-à-vis de composés herbicides ayant pour cible une enzyme impliquée dans l'une des étapes métaboliques de transformation du préphénate en L-tyrosine, **caractérisé en ce que** l'on transforme lesdites plantes avec un gène codant une enzyme préphénate dehydrogénase de manière à ce qu'elles l'expriment dans leurs tissus.

15. Procédé selon la revendication 12, **caractérisé en ce qu'**il s'applique à la production de plantes tolérantes vis-à-vis de composés herbicides ayant pour cible l'enzyme arogénate déhydrogénase

16. Procédé selon la revendication 15, **caractérisé en ce qu'**il s'applique à la production de plantes tolérantes vis-à-vis de composés herbicides ayant pour cible l'enzyme préphénate aminotransférase

17. Procédé selon l'une des revendications 14 à 16, **caractérisé en ce que** l'enzyme prephenate dehydrogénase provient d'une levure

18. Procédé selon l'une des revendications 14 à 16, **caractérisé en ce que** l'enzyme prephenate dehydrogénase provient d'un champignon

19. Procédé selon l'une des revendications 14 à 16, **caractérisé en ce que** l'enzyme prephenate dehydrogénase provient d'une bactérie
